# EUROPEAN PATENT APPLICATION

(11) **EP 3 560 942 A1**
(43) Date of publication of application: **30.10.2019**
(21) Application number: 19171251.2
(22) Date of filing: 26.04.2019
(51) Int. Cl.: C07H 15/203, C07H 1/00

(54) **XYLOSE DERIVATIVES AND PROCESS FOR PREPARATION THEREOF**

(30) Priority: 26.04.2018 US 201815963154
(71) Applicant: National Taipei University of Technology, Taipei 10608 (TW)
(72) Inventor: HWA, Kuo-Yuan, 10608 Taipei (TW)
(74) Representative: Becker Kurig Straus

(57) **Abstract**

Disclosure of the present invention relates to a method for synthesis of a xylose derivative, which comprises protecting a xylose with a protective group, followed by incorporating to a halogen atom as a leaving group; removing the protective groups and using water-soluble ligands to carry out a Suzuki cross-couplings reaction with a palladium catalyst in a water solution. Ten new xylose derivatives as obtained by the method are also provided.

## Description

### FIELD OF THE INVENTION

The present invention relates to a new method for synthesis of xylose derivatives and new xylose derivatives as prepared therefrom.

### BACKGROUND OF THE INVENTION

Green chemistry is defined as the environmentally friendly design of chemical products and processes. It is a philosophy of chemical research and engineering that encourages the development of products and processes that minimize the use and generation of hazardous substances. Relevant examples include reducing toxic waste, using renewable raw materials, enabling ambient-condition reactions to lower energy consumption, using smaller amounts of catalyst, and enabling high-percentage recovery of the catalyst, etc. Green chemistry seeks to reduce and prevent pollution at its source and applies to biochemistry, organic chemistry, inorganic chemistry, physical chemistry, analytical chemistry, and even carbohydrate chemistry. Most commonly, it focuses on industrial applications such as minimizing hazardous waste and maximizing efficiency.

Recently, the development of water-soluble homogeneous catalysts contributes to economical green chemistry. Since water-soluble catalysts are easy to separate from the organic phase, they avoid the use of organic solvents and are cost effective and environmentally friendly. Normally, a green chemistry method needs the following features: (1) use of non-toxic catalysts and solvents; and (2) use of renewable resources to synthesize chemical compounds.

In recent years, saccharide chemistry has become well established in medical applications such as biological materials and drug syntheses. Saccharide drugs are used for the treatment of cancer, diabetes, AIDS, influenza, bacterial infections, and rheumatoid arthritis. Remarkable research has also been done on saccharide vaccines in recent years. However, most of the saccharide drugs originate from nature such as the polysaccharides and glycosides of plants, animals, and microorganisms.

Xylose is a sugar first isolated from wood, is classified as a monosaccharide of the aldopentose type. It is derived from hemicellulose, one of the main constituents of biomass. It was reported that the acid-catalysed degradation of hemicellulose gives furfiral. In addition, since xylose does not contain calories, it can be used as diet food. Xylose and xylose derivatives may be applied in various fields. Particularly, xylose and its derivatives may be applied in green chemistry industry.

However, it is desirable to develop an economical and efficient method to obtain various xylose derivatives through green chemistry method.

### SUMMARY OF THE INVENTION

Accordingly, the present invention provides a new method for synthesis of a xylose derivative in the aqueous phase in the presence of organometallic catalysts. Through the method, it is easy to modify xylose; to obtain new xylose derivatives from nature. Since the catalyst can be recycled and water is used as the solvent, the reaction is environmentally friendly and economical. Xylose can be modified to provide various functional groups, which may affect the organism and/or enable its application in biological materials.

In one aspect, the present invention provides a xylose derivative having the general formula I: 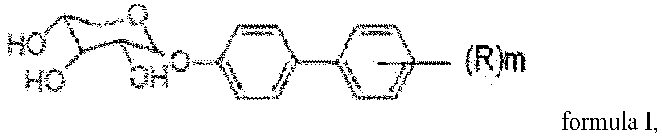 wherein the m for (R)m is an integer from 1 to 4; R is CH₃, OCH₃, or a halogen atom..

In the embodiment of the invention, the xylose derivative is one selected from the group consisting of the following: 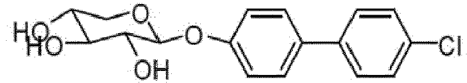 2-(4'-chlorobiphenyl-4-yloxy) tetrahydro-2*H*-pyran-3,4,5-triol (Compound 1); 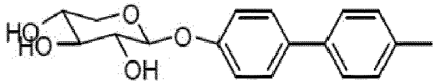 2-(4'-methylbiphenyl-4-yloxy) tetrahydro-2*H*-pyran-3,4,5-triol (Compound 2); 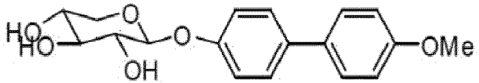 2-(4'-methoxybiphenyl-4-yloxy) tetrahydro-2*H*-pyran-3,4,5-triol (Compound 3); 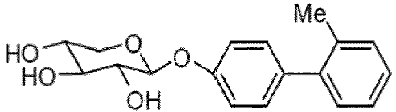 2-(2'-methylbiphenyl-4-yloxy)tetrahydro-2*H*-pyran-3,4,5-triol (Compound 4); 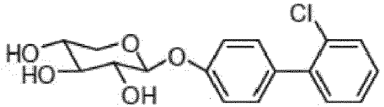 2-(2'-chlorobiphenyl-4-yloxy)tetrahydro-2*H*-pyran-3,4,5-triol (Compound 5); 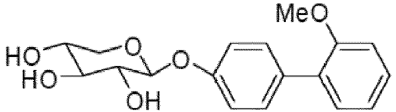 2-(2'-methoxybiphenyl-4-yloxy) tetrahydro-2*H*-pyran-3,4,5-triol (Compound 6); 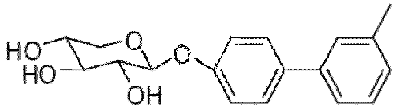 2-(3'-methylbiphenyl-4-yloxy) tetrahydro-2*H*-pyran-3,4,5-triol (Compound 7); 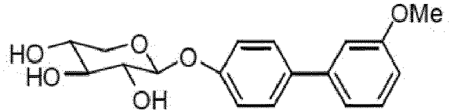 2-(3'-methoxybiphenyl-4-yloxy) tetrahydro-2*H*-pyran-3,4,5-triol (Compound 8); 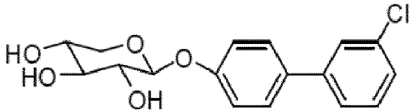 2-(3'-chlorobiphenyl-4-yloxy) tetrahydro-2*H*-pyran-3,4,5-triol (Compound 9); and 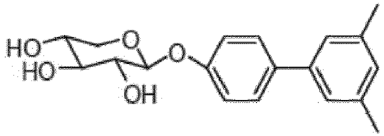 2-(3',5'-dimethylbiphenyl-4-yloxy)tetrahydro-2*H*-pyran-3,4,5-triol (Compound 10).

In another aspect, the present invention provides a method of synthesis of the xylose derivatives according to the present invention. The method comprises the steps of:
protecting a xylose with a protective group, followed by incorporating to a halogen atom as a leaving group;
removing the protective group and using a water-soluble ligand to carry out Suzuki cross-couplings reaction with a palladium catalyst in a water solution to synthesize the derivative.

In one example of the invention, the halogen atom is an iodine atom.

In one example of the invention, the water-soluble ligand is recycled.

In one example of the invention, the palladium catalyst is recycled.

In one example of the invention, the palladium catalyst is PdCl₂(NH₃)₂, and the water-soluble ligand is a cationic 2,2'-bipyridyl ligand.

In one example of the invention, a cationic 2,2'-bipyridyl ligand is dissolved in water to form a solution A; and PdCl₂(NH₃)₂ is dissolved in water to form a solution B; and adding the solution A to the solution B to generate a turbid solution that eventually cleared to obtain a water-soluble palladium catalyst.

In one example of the invention, the Suzuki cross-couplings reaction is carried out at a temperature no higher than 100°C.

### BRIEF DESCRIPTION OF THE DRAWINGS

The foregoing summary, as well as the following detailed description of the invention, will be better understood when read in conjunction with the appended drawing.

In the drawings:
Figure 1A shows the ¹H NMR spectrum of the starting material 2-(4-iodophenoxy)tetrahydro-2*H*-pyran-3,4,5-triol, which features the peaks for the hydrogen atoms in the tetrahydrofuran and hydroxyl moieties in the range of δ∼3.00-5.50 ppm, wherein the peak at 2.0 ppm disappears rather than the peaks corresponding to the benzene ring.
Figure 1B shows the ¹³C NMR spectrum of 2-(4-iodophenoxy)tetrahydro-2*H*-pyran-3,4,5-triol, which features the solvent peak at ∂ 43.5 ppm and the hydroxyl group at the C1 position of xylose appears at δ2.0 ppm.
Figure 2A shows the ¹H NMR spectrum of 2-(4'-chlorobiphenyl-4-yloxy) tetrahydro-2*H*-pyran-3,4,5-triol, , which features the peaks in the range of δ∼3.00-5.50 ppm correlate with the hydrogen atoms of the hydroxyl and tetrahydrofuran moieties.
Figure 2B shows the enlargement of the ¹H NMR spectrum of 2-(4'-chlorobiphenyl-4-yloxy)tetrahydro-2*H*-pyran-3,4,5-triol
Figure 2C shows the ¹³C NMR spectrum of 2-(4'-chlorobiphenyl-4-yloxy)tetrahydro-2*H*-pyran-3,4,5-triol.
Figure 2D shows the enlargement of ¹³C NMR spectrum of 2-(4'-chlorobiphenyl-4-yloxy)tetrahydro-2*H*-pyran-3,4,5-triol.
Figure 3A shows the ¹H NMR spectrum of 2-(4'-methylbiphenyl-4-yloxy) tetrahydro-2*H*-pyran-3,4,5-triol, which features the peaks in the range of δ∼3.00-5.50 ppm correlate with the hydrogen atoms of the hydroxyl and tetrahydrofuran moieties.
Figure 3B shows the enlargement of the ¹H NMR spectrum of 2-(4'-methylbiphenyl-4-yloxy)tetrahydro-2*H*-pyran-3,4,5-triol.
Figure 3C shows the ¹³C NMR spectrum of 2-(4'-methylbiphenyl-4-yloxy)tetrahydro-2*H*-pyran-3,4,5-triol.
Figure 4A shows the ¹H NMR spectrum of 2-(4'-methoxybiphenyl-4-yloxy) tetrahydro-*2H*-pyran-3,4,5-triol, which features the peaks in the range of δ∼3.00-5.50 ppm correlate with the hydrogen atoms of the hydroxyl and tetrahydrofuran moieties.
Figure 4B shows the ¹³C NMR spectrum of 2-(4'-methoxybiphenyl-4-yloxy) tetrahydro-2*H*-pyran-3,4,5-triol.
Figure 5A shows the ¹H NMR spectrum of 2-(2'-methylbiphenyl-4-yloxy) tetrahydro-*2H*-pyran-3,4,5-triol. Peaks in the range of δ∼3.00-5.50 ppm correlate with the hydrogen atoms of the hydroxyl and tetrahydrofuran moieties.
Figure 5B shows the enlargement of the ¹H NMR spectrum of 2-(2'-methylbiphenyl-4-yloxy) tetrahydro-*2H*-pyran-3,4,5-triol.
Figure 5C shows the ¹³C NMR spectrum of 2-(2'-methylbiphenyl-4-yloxy) tetrahydro-2*H*-pyran-3,4,5-triol.
Figure 6A shows the ¹H NMR spectrum of 2-(2'-chlorobiphenyl-4-yloxy)tetrahydro-*2H*-pyran-3,4,5-triol, which features the peaks in the range of δ∼3.00-5.50 ppm correlating with the hydrogen atoms of the hydroxyl and tetrahydrofuran moieties.
Figure 6B shows the ¹³C NMR spectrum of 2-(2'-chlorobiphenyl-4-yloxy)tetrahydro-*2H*-pyran-3,4,5-triol.
Figure 7A shows the ¹H NMR spectrum of 2-(2'-methoxybiphenyl-4-yloxy) tetrahydro-2*H*-pyran-3,4,5-triol, which features the peaks in the range of δ∼3.00-5.50 ppm correlating with the hydrogen atoms of the hydroxyl and tetrahydrofuran moieties.
Figure 7B shows the enlargement of the ¹H NMR spectrum of 2-(2'-methoxybiphenyl-4-yloxy) tetrahydro-2*H*-pyran-3,4,5-triol.
Figure 7C shows the ¹³C NMR spectrum of 2-(2'-methoxybiphenyl-4-yloxy) tetrahydro-2*H*-pyran-3,4,5-triol.
Figure 8A shows the ¹H NMR spectrum of 2-(3'-methylbiphenyl-4-yloxy) tetrahydro-*2H*-pyran-3,4,5-triol, which features the peaks in the range of δ∼3.00 -5.50 ppm correlating with the hydrogen atoms of the hydroxyl and tetrahydrofuran moieties.
Figure 8B shows the enlargement of the ¹H NMR spectrum of 2-(3'-methylbiphenyl-4-yloxy) tetrahydro-*2H*-pyran-3,4,5-triol.
Figure 8C shows the ¹³C NMR spectrum of of 2-(3'-methylbiphenyl-4-yloxy) tetrahydro-*2H*-pyran-3,4,5-triol.
Figure 9A shows the ¹H NMR spectrum of 2-(3'-methoxybiphenyl-4-yloxy)tetrahydro-*2H*-pyran-3,4,5-triol, which features the peaks in the range of δ∼3.00-5.50 ppm correlating with the hydrogen atoms of the hydroxyl and tetrahydrofuran moieties.
Figure 9B shows the enlargement of the ¹H NMR spectrum of 2-(3'-methoxybiphenyl-4-yloxy)tetrahydro-2*H*-pyran-3,4,5-triol.
Figure 9C shows the ¹³C NMR spectrum of 2-(3'-methoxybiphenyl-4-yloxy)tetrahydro-*2H*-pyran-3,4,5-triol.
Figure 10A shows the ¹H NMR spectrum of 2-(3'-chlorobiphenyl-4-yloxy) tetrahydro-*2H*-pyran-3,4,5-triol, which features the peaks in the range of δ∼3.00-5.50 ppm correlating with the hydrogen atoms of the hydroxyl and tetrahydrofuran moieties.
Figure 10B shows the enlargement of the ¹H NMR spectrum of 2-(3'-chlorobiphenyl-4-yloxy) tetrahydro-*2H*-pyran-3,4,5-triol.
Figure 10C shows the ¹³C NMR spectrum of 2-(3'-methoxybiphenyl-4-yloxy)tetrahydro-*2H*-pyran-3,4,5-triol.
Figure 11A shows the ¹H NMR spectrum of 2-(3',5'-dimethylbiphenyl-4-yloxy)tetrahydro-2*H*-pyran-3,4,5-triol, which features the peaks in the range of δ∼3.00-5.50 ppm correlating with the hydrogen atoms of the hydroxyl and tetrahydrofuran moieties.
Figure 11B shows the ¹³C NMR spectrum of 2-(3',5'-dimethylbiphenyl-4-yloxy)tetrahydro-2*H*-pyran-3,4,5-triol.

### DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereof known to those skilled in the art.

As used herein, the term "a halogen atom" refers to one atom selected from one group in the periodic table consisting of five chemically related elements: fluorine (F), chlorine (Cl), bromine (Br), iodine (I), and astatine (At).

The present invention provides a method for synthesis of a xylose derivative in the aqueous phase in the presence of a water-soluble palladium catalyst, which provides a friendly and economical green chemistry method for synthesis of xylose derivatives.

The technologies and materials involved in the present invention are illustrated below.

### Solvent Purification

On a distillation apparatus under a nitrogen atmosphere, magnesium sulfate was added to dichloromethane in the distilling flask and the temperature was set at ∼50 °C. The distillate was collected in a flask containing dried molecular sieves.

### Free-of-Water and Air Process

If the reactions are sensitive to water and air, all experimental processes including reaction, extraction, drying, concentration and column chromatography must be carried out in the absence of air under nitrogen conditions. Therefore, the solvents and chemicals must be free of water and air before use and the liquids must be transferred using syringes and flex needles (Teflon). The solution is concentrated using a rotary vacuum concentrator and a vacuum motor.

### Reaction Scheme

According to the invention, the scheme of the total reactions in the method for synthesis of a xylose derivative is given below:

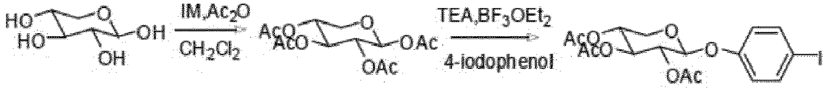

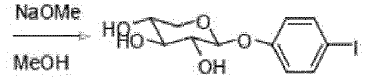

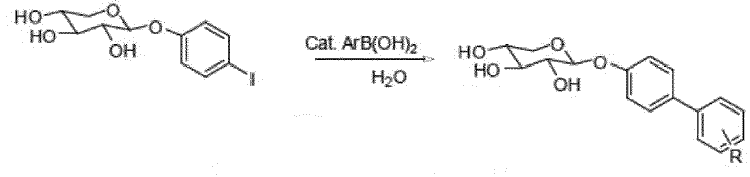

### Acetylation Reaction

In one example of the invention, the scheme of acetylation reaction is given below:

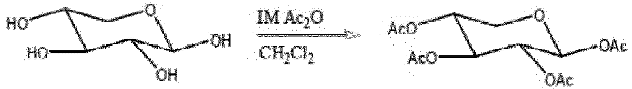

A solution of 0.75 g of xylose (5 mmol), 2.84 mL (30 mmol) of acetic anhydride, and 2.04 g (30 mmol) of imidazole in 700 mL of dichloromethane was placed in an ice bath for 30 min and then stirred for 12 hours. The reaction was then quenched by the addition of methanol and stirred in ice for 30 min. The mixture was diluted with dichloromethane, adjusted to a neutral pH using HCl, and extracted with Et₂O. The usual work-up and column chromatography afforded acetylated xylose (1.50 g, 94%) as a white solid.

### Phenol Derivation Reaction

In one example of the invention, the scheme of phenol derivation reaction is given below (Lee et al., CARBOHYDRATE CHEMISTRY. 20(6): 503-506, 2001):

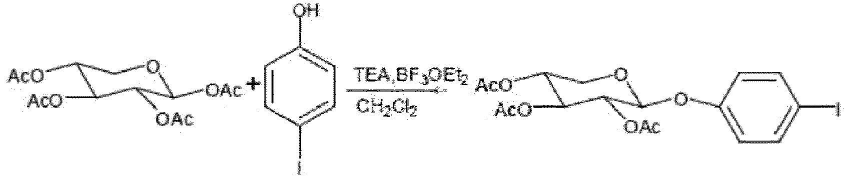

A solution of 1.59 g (5 mmol) of acetylated xylose, 1.29 g (7.5 mmol) of 4-bromophenol, 0.7 mL (5 mmol) of TEA, and 0.87 mL (12.5 mmol) in 30 mL of dichloromethane under a nitrogen atmosphere was stirred for 24 hours to convert the 4-bromophenol to 4-iodophenol. The mixture was diluted with 20 mL of saturated aqueous NaHCO_{3(aq)} and extracted with Et₂O (30 mL × 3). The usual work-up and column chromatography afforded the product (1.29 g, 54%) as a white solid.

### Deacetylation Reaction

In one example of the invention, the scheme of deacetylation reaction is given below (Will et al., J. Phys CHEM. B, 103:8067, 1999):

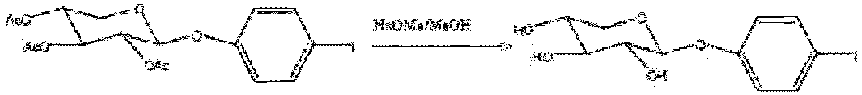

A solution of 0.478 g (1 mmol) of starting material and 0.33 g (6 mmol) of sodium methoxide in 20 mL of methanol under a constant pressure of air was stirred for 8 hours. The mixture was adjusted to a neutral pH by the addition of HCl and then extracted with Et₂O (30 mL × 3). The usual work-up and column chromatography afforded the product (0.32 g, 91%) as a white solid.

### Synthesis of Cationic 2,2-Bipyridyl Ligand

In the embodiment of the invention, the water-soluble ligand is a cationic 2,2-bipyridyl ligand, which may be synthesis according to the scheme below (Xue et al., Carbohydrate Research 344:1646-1653, 2009):

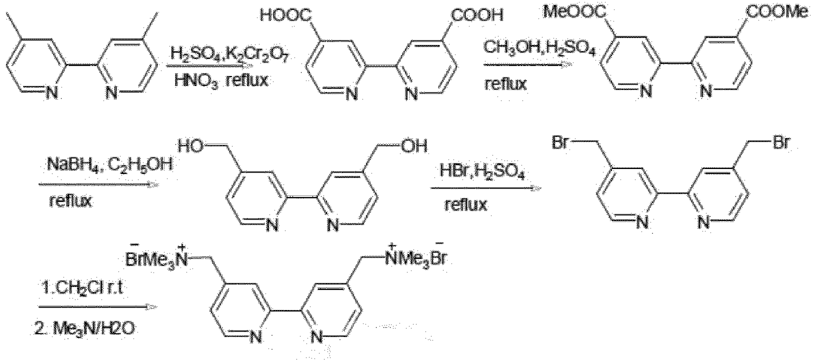

The following ligands may be used in the present invention:
4,4'-dicarboxyl-2,2'-bipyridine;
4,4'-dimethoxycarbonyl-2,2'-bipyridine;
4,4'-bis(hydroxymethyl)-2,2'-bipyridine;
4,4'-bis(bromomethyl)-2,2'-bipyridine; or
cationic 2,2'-bipyridine ligand.

The syntheses of the above mentioned ligands are given below.

### (1) Synthesis of 4,4'-dicarboxyl-2,2'-bipyridine

The scheme of the synthesis of 4,4'-dicarboxyl-2,2'-bipyridine is given below:

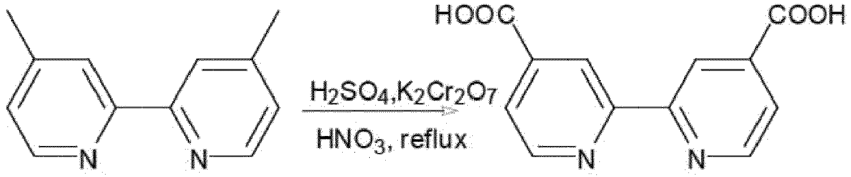

4,4'-Dimethyl-2,2'-bipyridine (5 g) was dissolved in 125 mL of H₂SO₄ (98%). K₂Cr₂O₇ was added and the temperature was maintained between ∼70-80 °C. The reaction was quenched when the solution changed from yellow to black. A yellow solid was obtained after filtration and concentration. This yellow solid was dissolved in 170 mL of HNO_{3(aq)} (50%) and refluxed for 4 hours. The solution was then diluted to 1 L, filtered, and washed with water and acetone. After concentrating the solution, the product was obtained (6.12 g, 93%) as a white solid and identified using ¹H NMR spectroscopy.

### (2) Synthesis of 4,4'-dimethoxycarbonyl-2,2'-bipyridine

The scheme of synthesis of 4,4'-dimethoxycarbonyl-2,2'-bipyridine is given below:

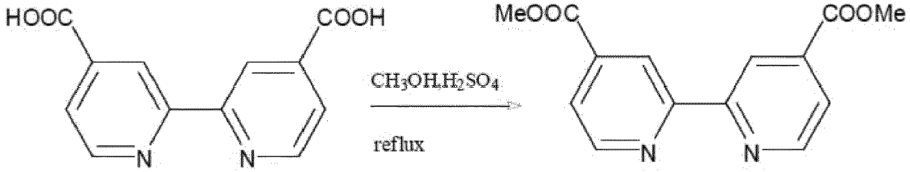

4,4'-Dicarboxyl-2,2'-bipyridine (6.12 g) was dissolved in 115 mL of MeOH_{(aq)} and 15 mL of H₂SO_{4(aq)}. The solution was refluxed at ∼90-100 °C for 24 hours after which the reaction was quenched with 300 mL of DDW. NaOH_{(aq)} was added until the pH reached ∼8.0. The organic layer was extracted using dichloromethane (2 × 50 mL) and dried with MgSO₄. It was then filtered and concentrated in a vacuum evaporator to yield the solid product (5.9 g, 87%). The compound was identified using ¹H NMR spectroscopy.

### (3) Synthesis of 4,4'-bis(hydroxymethyl)-2,2-bipyridine

The scheme of the synthesis of 4,4'-bis(hydroxymethyl)-2,2'-bipyridine is given below:

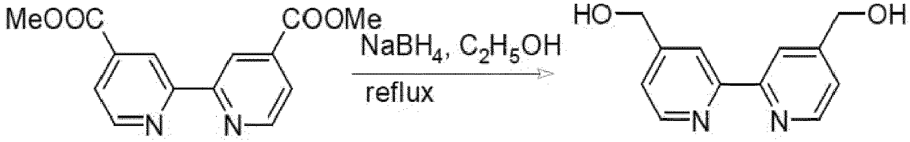

4,4'-Dimethoxycarbonyl-2,2'-bipyridine (5.9 g) was dissolved in pure EtOH to which sodium borohydride (12 g) was added. The mixture was refluxed for 3 hours at ∼90-100 °C. After cooling to room temperature, saturated NH₄Cl(aq) was added followed by 300 mL of DDW. The solution was then extracted using ethyl acetate (5 × 250 mL), dried with MgSO₄, filtered and then concentrated in a vacuum evaporator to obtain the desired solid (4.46 g, 95%). The product was identified using ¹H NMR spectroscopy.

### (4) Synthesis of 4,4-bis(bromomethyl)-2,2'-bipyridin

The scheme of the synthesis of 4,4'-bis(bromomethyl)-2,2'-bipyridine is given below:

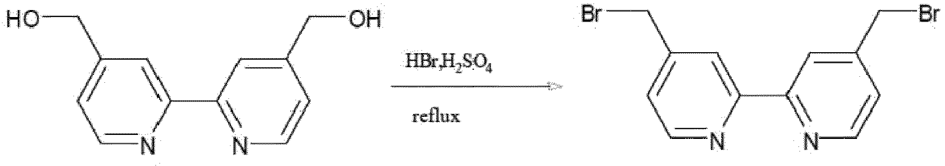

4,4'-Bis(hydroxymethyl)-2,2'-bipyridine (4.46 g) was dissolved in 30 mL of HBr (48%) and 10 mL of concentrated sulfuric acid. The solution was then refluxed for 6 hours at 110 °C. When the solution changed from light yellow to golden, the heat was removed and 100 mL of DDW was added to quench the reaction. The pH of the solution was adjusted to 7.0 using NaOH(aq), which caused the precipitation of a pink compound. The precipitate was collected via filtration, washed with DDW, and dried under vacuum. The liquid portion was extracted with ethyl acetate (2 × 50 mL), dried with MgSO₄, filtered, and concentrated in a vacuum evaporator. The desired product (4.39 g, 88%) was obtained and identified via ¹H NMR spectroscopy.

### (5) Synthesis of cationic 2,2'-bipyridine ligand

The Scheme of the synthesis of cationic 2,2'-bipyridine ligand is given below:

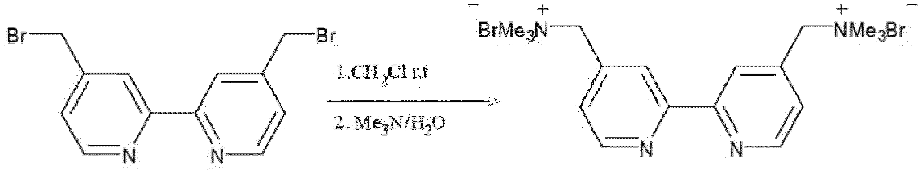

4,4'-Bis(bromomethyl)-2,2'-bipyridine (0.5 g) was dissolved in dichloromethane and 20 mL of trimethylamine (50%) was added. The mixture was left to react for 24 hours at room temperature. A freeze dryer was used for 2 days to remove any water and obtain the desired product.

### Preparation of the Water-Soluble Palladium Catalyst

In one embodiment of the invention, the palladium catalyst is PdCl₂(NH₃)₂ which is also known as Pd(NH₃)₂Cl₂. The water-soluble palladium catalyst may be prepared according to the scheme below:

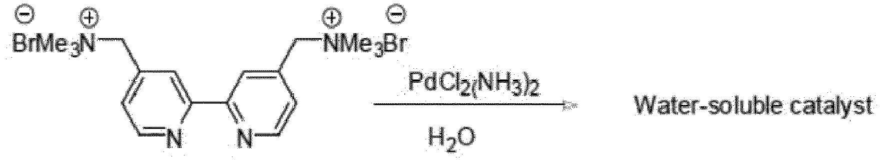

The cationic 2,2'-bipyridyl ligand (0.2 mmol) was dissolved in 5 mL of water in a round bottom flask to form solution A. PdCl₂(NH₃)₂ (0.2 mmol) was dissolved 10 mL of water in a separate round bottom flask to form solution B. Solution A was slowly added to solution B under ultrasonic vibration to generate a turbid solution that eventually cleared. Finally, 5 mL of water was used to fully transfer solution B into solution A until the solution was completely clear. The resultant water-soluble palladium catalyst has a concentration of 0.2 mmol/20 mL, i.e., 0.01 mmol/mL. This solution was diluted to prepare aqueous solutions of palladium catalyst with concentrations of 0.001 and 0.0001 mmol/mL.

### Suzuki Cross-Coupling Reaction

The scheme of Suzuki cross-coupling reaction is given below (Wu et al., Tetrahedron Letters. 47: 9267-927, 2006):

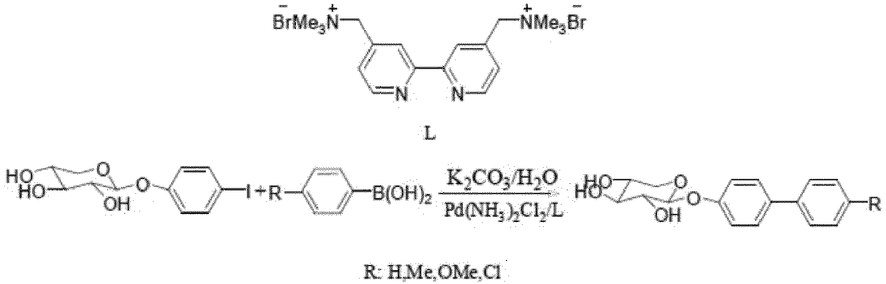

A sealable tube equipped with a magnetic stirrer bar was charged with the starting material (1 mmol), arylboronic acid (1.5 mmol), K₂CO₃ (2 mmol), and H₂O (2 mL). In the case of styrene, the addition of TBAB (1 mmol) was required. After the addition of the Pd(NH₃)₂Cl₂/L aqueous solution, the tube was sealed under air using a Teflon-coated screw cap. The reaction vessel was then placed in an oil bath at 100°C or no higher than 100°C for 24 hours. After cooling the reaction mixture to room temperature, the aqueous solution was extracted with ethyl acetate, the organic phase was dried over MgSO₄, and the solvent was removed under vacuum. Column chromatography on silica gel afforded the desired product.

In one example of the invention, the Suzuki cross-couplings reaction is carried out at a temperature no higher than 100°C.

### Recycling of Water-Soluble Palladium Catalyst

The scheme of the recycling of water-soluble palladium catalyst is given below (Wu et al., Tetrahedron Letters. 47: 9267-9270, 2006):

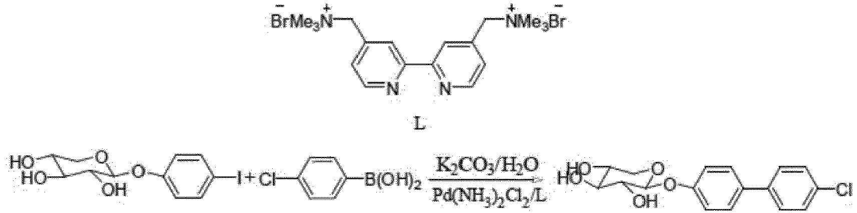

A sealable tube equipped with a magnetic stirrer bar was charged with the starting material (1 mmol), 4-chlorophenylboronic acid (1.5 mmol), K₂CO₃ (2 mmol), and H₂O (2 mL). In the case of styrene, the addition of TBAB (1 mmol) was required. After the addition of the Pd(NH₃)₂Cl₂/L aqueous solution, the tube was sealed under air using a Teflon-coated screw cap. The reaction vessel was then placed in an oil bath at 100 °C for 24 hours. After cooling the reaction mixture to room temperature, the aqueous solution was extracted with ethyl acetate, the organic phase was dried over MgSO₄, and the solvent was removed under vacuum. Column chromatography on silica gel afforded the desired product. After the reaction, the aqueous reaction mixture was washed with ethyl acetate three times with vigorous stirring and the organic product was isolated from the combined organic phase according to a previously described procedure. The residual aqueous solution was then charged with 4-chlorophenylboronic acid and K₂CO₃ for the next reaction; in the case of styrene, the addition of TBAB for the first run was required.

### Protective Groups

Any protective groups commonly used in the art may be used in this present invention. For instance, acyl protective groups commonly used in carbohydrate chemistry, alkyl ethers used in ether protection to identify and analyze the polysaccharides, cyclic acetals or cycloketals in acetal ketone protection, protective groups for amino moieties such as *N*-acetyl and phthalyl and other protective groups. Some common protective groups are listed below:
(1) Esters: 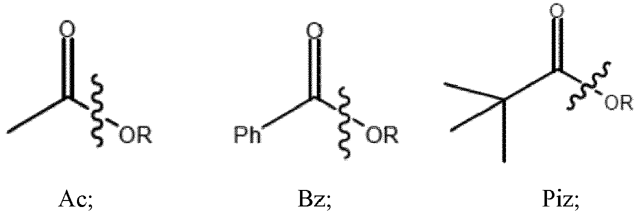
(2) Ethers: 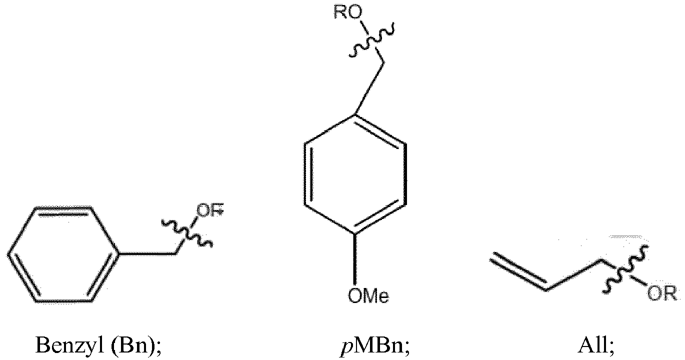
(3) Acetals (ketals): 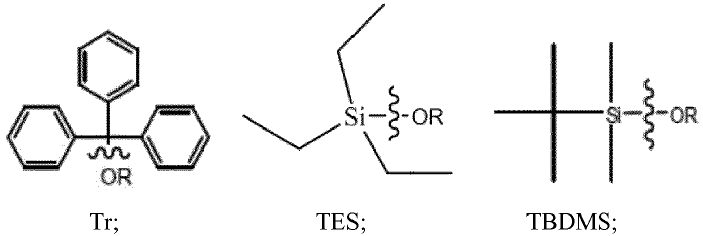
(4) Aminos:

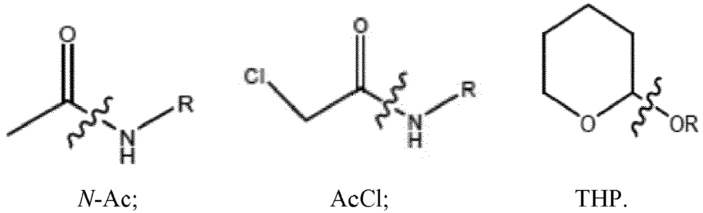

According to the invention, new xylose derivatives have been synthesized, which have the general formula I 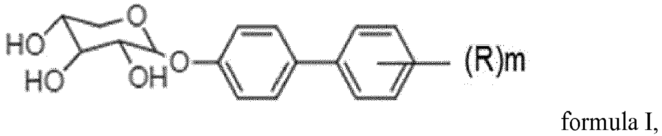 wherein the m for (R)m is an integer from 1 to 4; R is CH₃, OCH₃, or a halogen atom.

In the examples of the invention, the xylose derivative is one selected from the group consisting of the following: 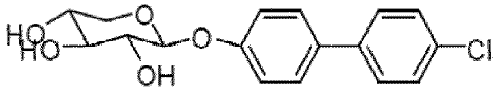 2-(4'-chlorobiphenyl-4-yloxy) tetrahydro-2*H*-pyran-3,4,5-triol (Compound 1); 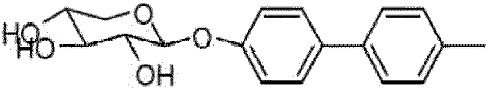 2-(4'-methylbiphenyl-4-yloxy) tetrahydro-2*H*-pyran-3,4,5-triol (Compound 2); 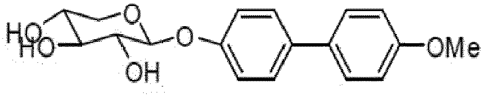 2-(4'-methoxybiphenyl-4-yloxy) tetrahydro-2*H*-pyran-3,4,5-triol (Compound 3); 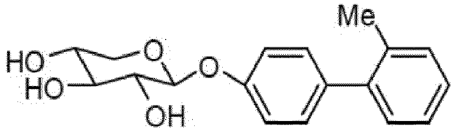 2-(2'-methylbiphenyl-4-yloxy)tetrahydro-2*H*-pyran-3,4,5- triol (Compound 4); 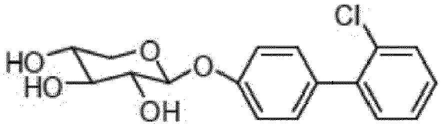 2-(2'-chlorobiphenyl-4-yloxy)tetrahydro-*2H*-pyran-3,4,5-triol (Compound 5); 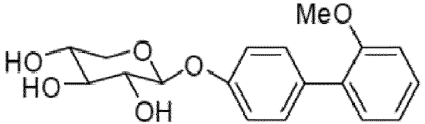 2-(2'-methoxybiphenyl-4-yloxy) tetrahydro-2*H*-pyran-3,4,5-triol (Compound 6); 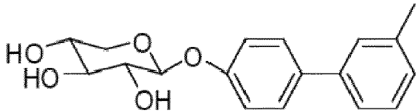 2-(3'-methylbiphenyl-4-yloxy) tetrahydro-2*H*-pyran-3,4,5-triol (Compound 7); 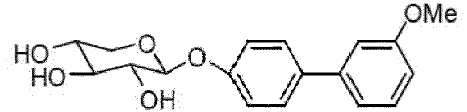 2-(3'-methoxybiphenyl-4-yloxy) tetrahydro-2*H*-pyran-3,4,5-triol (Compound 8); 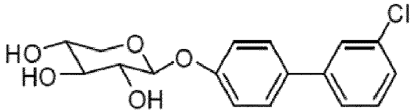 2-(3'-chlorobiphenyl-4-yloxy) tetrahydro-2*H*-pyran-3,4,5-triol (Compound 9); and 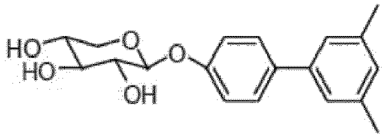 2-(3',5'-dimethylbiphenyl-4-yloxy)tetrahydro-*2H*-pyran-3,4,5-triol (Compound 10).

The present invention is further illustrated by the following examples, which are provided for the purpose of demonstration rather than limitation

### Examples

### Example 1 Preparation of Starting Material

For synthesis of xylose derivatives, 2-(4-iodophenoxy) tetrahydro-2*H*-pyran-3,4,5-triol as shown below is used as a starting material:

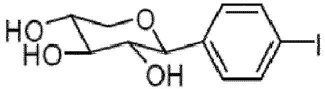

The scheme of the total reactions of the starting material is given below:

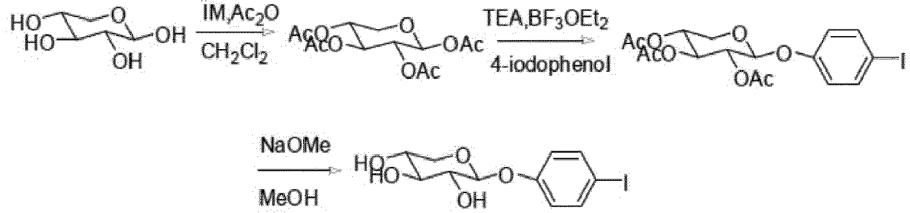

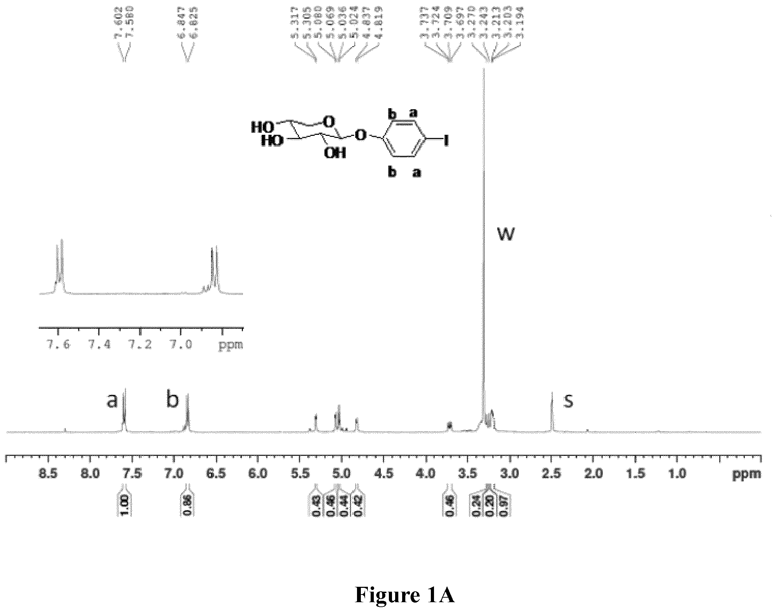

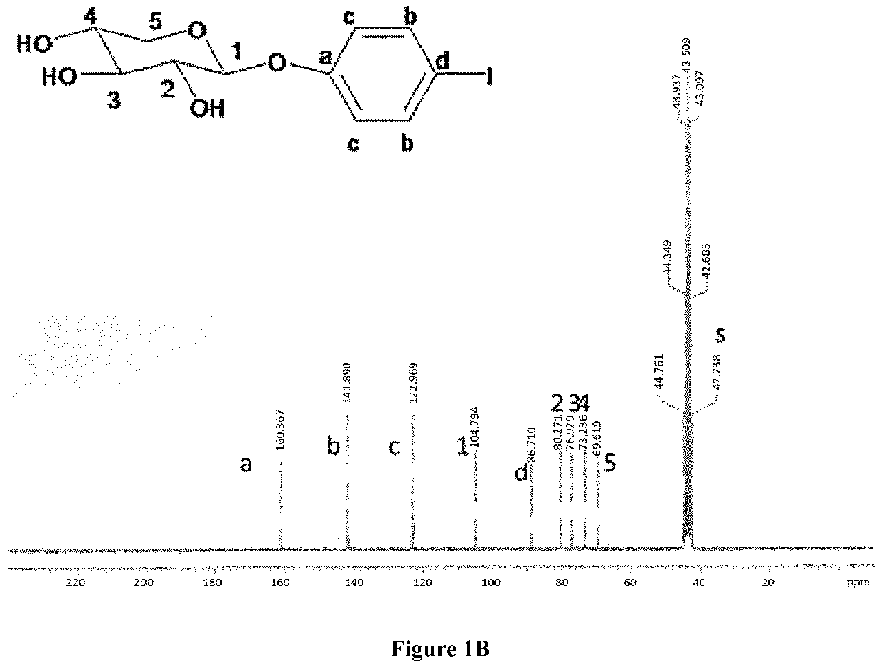

The crude product was purified by column chromatography on silica gel and gave the product as a white power in a 45.9% yield. Figure 1A shows the ¹H NMR spectrum of 2-(4-iodophenoxy)tetrahydro-2*H*-pyran-3,4,5-triol, which features the peaks for the hydrogen atoms in the tetrahydrofuran and hydroxyl moieties in the range of δ∼3.00-5.50 ppm. Figure 1B shows the ¹³C NMR spectrum of 2-(4-iodophenoxy)tetrahydro-2*H*-pyran-3,4,5-triol, wherein the solvent peak appears at δ43.5 ppm and the hydroxyl group at the Cl position of xylose appears at δ2.0 ppm. However, the peak at δ2.0 ppm disappears in Figure 1A, rather than the peaks corresponding to the benzene ring. Therefore, it was confirmed that the expected product was obtained.

¹H NMR (400 MHz, CD₆SO): δ7.59 (d, *J* = 8.8 Hz, 2H), 6.84 (d, *J* = 8.8Hz, 2H). ¹³C NMR (50 MHz, CD₆SO): δ160.37, 141.89, 122.97, 104.79, 86.72, 80.27, 76.93, 73.24, 69.62. Mass Calcd for C₁₁H₁₃IO₄: 336.08.

### Preparation Examples for Syntheses of Xylose Derivatives

The starting material as obtained in Example 1 was used to synthesize xylose derivatives through a Suzuki cross-coupling reaction with a palladium catalyst in a water solution, the scheme of which is given below:

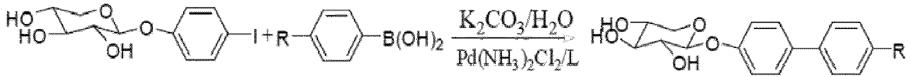

### Ar-B(OH)₂

The 10 xylose derivatives were synthesized and the purification and characterization thereof were given in the following examples. The results of the catalysis of xylose and arylboronic acids (Ar-B(OH)₂) are given in Table 1.

**Table 1 Results of the catalysis of xylose and arylboronic acids**

| Compound No. | Ar-B(OH)₂ | Structure of Product | Yield (%) | Weight (g) |
|---|---|---|---|---|
| Compound 1 | 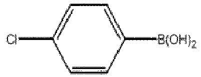 | 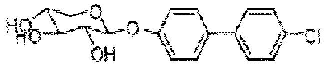 | 71.4 | 0.121 |
| Compound 2 | 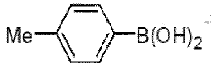 | 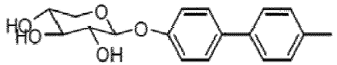 | 54.5 | 0.086 |
| Compound 3 | 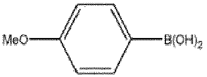 | 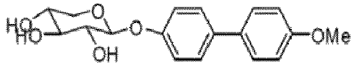 | 78.3 | 0.131 |
| Compound 4 | 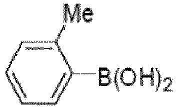 | 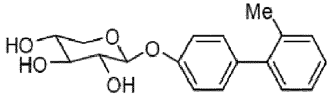 | 51.8 | 0.082 |
| Compound 5 | 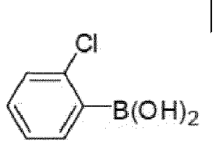 | 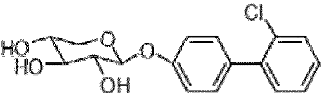 | 55.3 | 0.093 |
| Compound 6 | 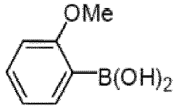 | 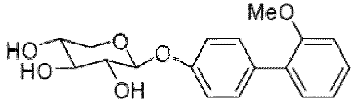 | 60.8 | 0.101 |
| Compound 7 | 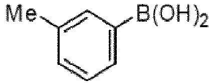 | 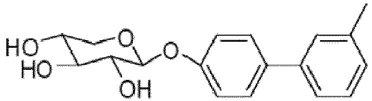 | 87.5 | 0.138 |
| Compound 8 | 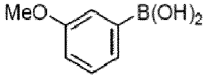 | 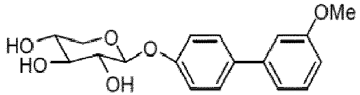 | 92.0 | 0.139 |
| Compound 9 | 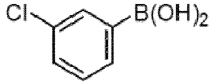 | 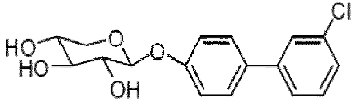 | 77.2 | 0.13 |
| Compound 10 | 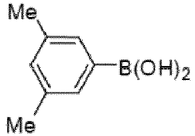 | 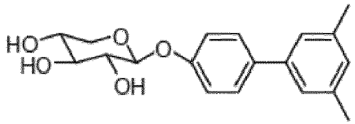 | 98.0 | 0.162 |

### Example 2

### 2-(4'-Chlorobiphenyl-4-yloxy) tetrahydro-2H-pyran-3,4,5-triol (Compound 1):

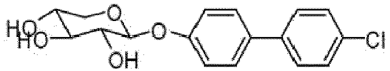

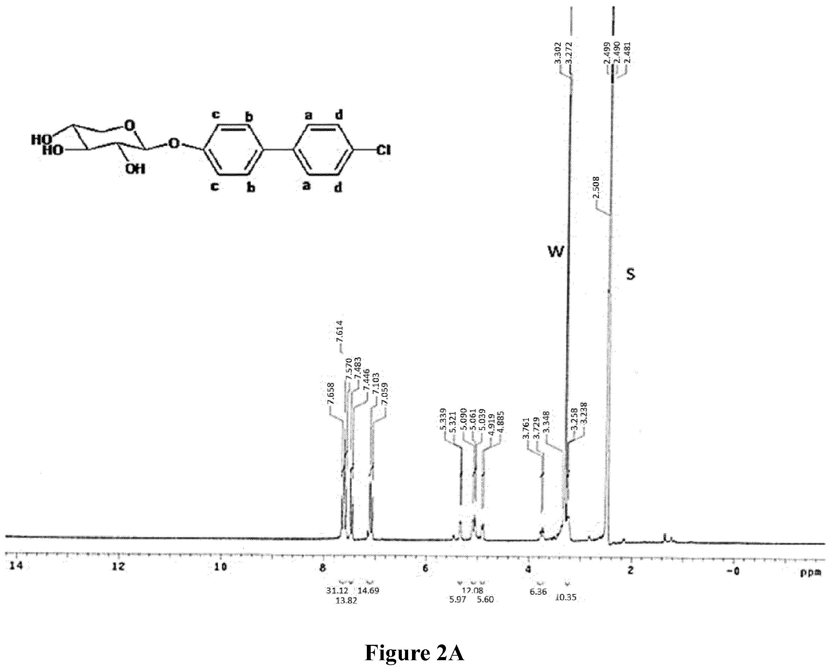

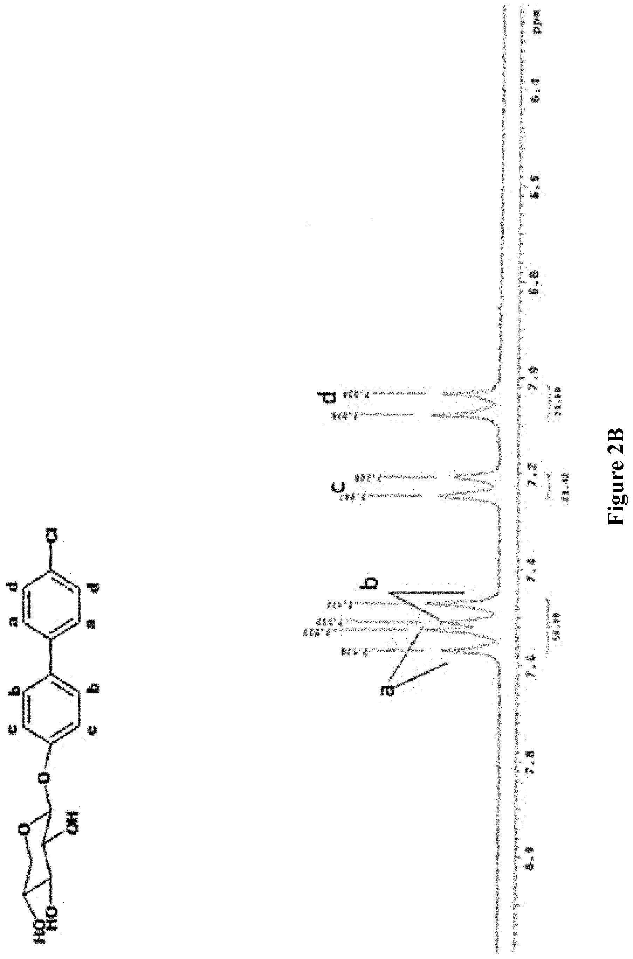

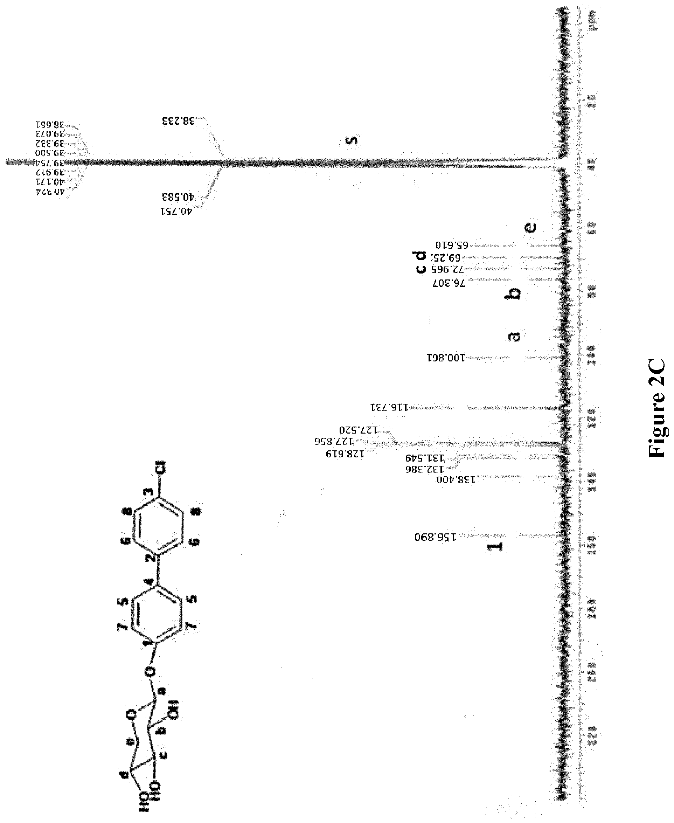

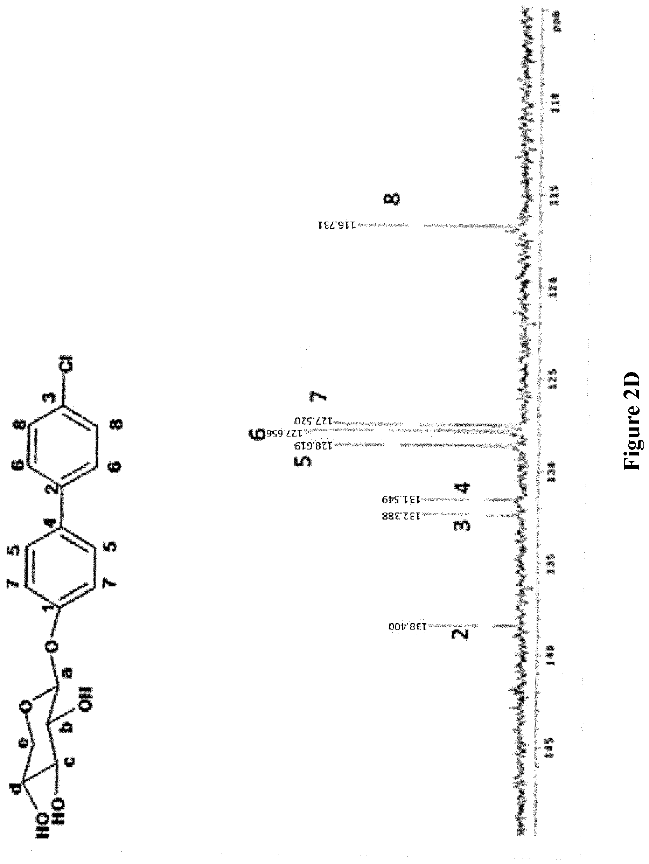

The crude product was purified by column chromatography on silica gel to yield the purified product as a white powder in a 79.2% yield. Figures 2A and 2B show the ¹H NMR spectra of 2-(4'-chlorobiphenyl-4-yloxy) tetrahydro-2*H*-pyran-3,4,5-triol. Figures 2C and 2D show the ¹³C NMR spectra of 2-(4'-chlorobiphenyl-4-yloxy) tetrahydro-2*H*-pyran-3,4,5-triol. In the spectra, the peaks in the range of δ∼3.00-5.50 ppm correlate with the hydrogen atoms of the hydroxyl and tetrahydrofuran moieties. Since there is iodine in the starting material, i.e., 2-(4-iodophenoxy)tetrahydro-2*H*-pyran-3,4,5-triol, the hydrogen peak shifts from δ7.71 to 7.59 ppm. As shown in Figures 2A-2D, it is confirmed that the desired product was obtained.

¹H NMR (200 MHz, CD₆SO): δ7.64 (d, *J* = 8.8Hz, 2H), 7.59 (d, *J* = 8Hz, 2H), 7.46(d, *J* = 8.6, 2H), 7.08(d, *J* = 8.8Hz, 2H). ¹³C NMR (50 MHz, CD₆SO): δ156.89, 138.4, 132.39, 131.55, 128.86, 127.86, 127.52, 116.73, 100.86, 76.3, 72.97, 69.26, 65.61. Mass Calcd for C₁₇H₁₈O₅: 302.12.

### Example 3

### 2-(4'-Methylbiphenyl-4-yloxy) tetrahydro-2H-pyran-3,4,5-triol (Compound 2):

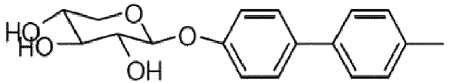

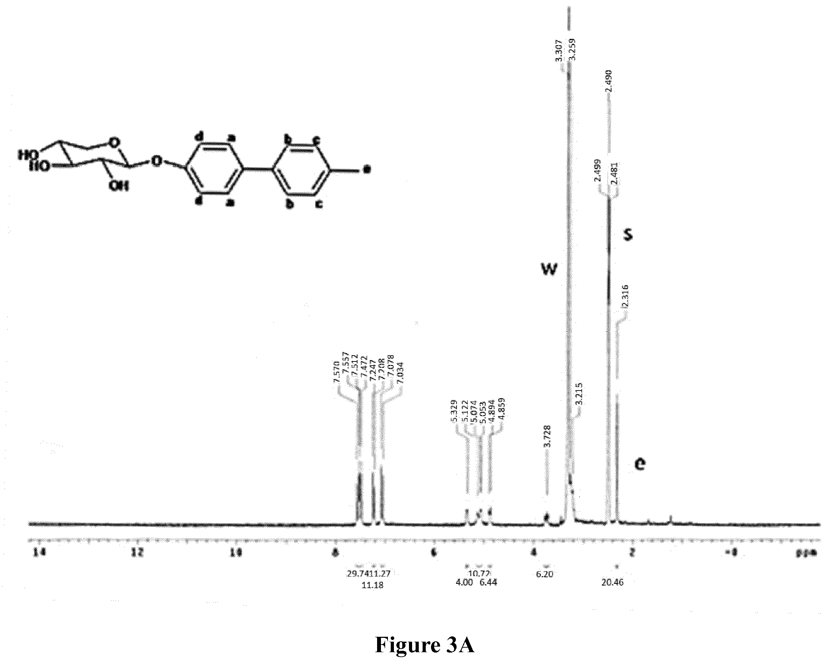

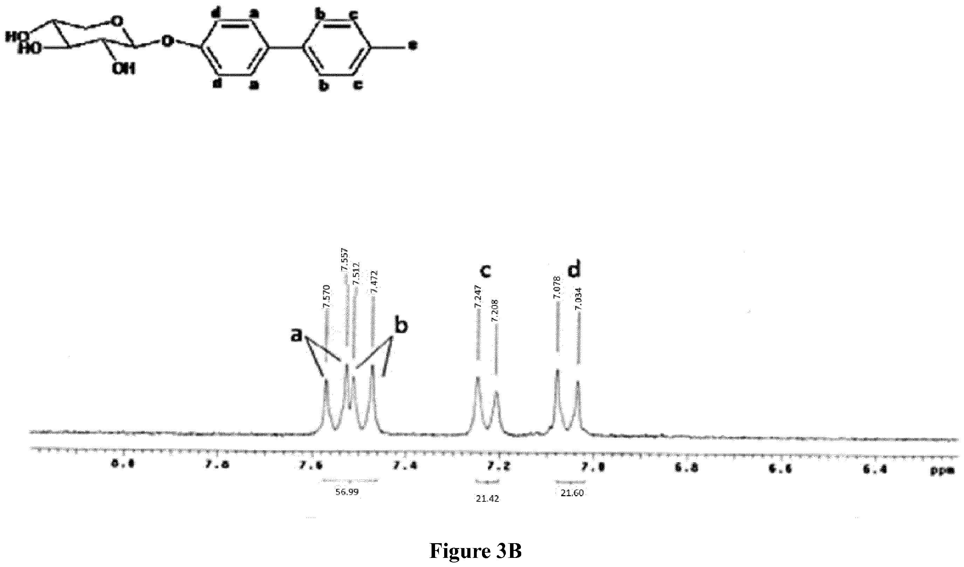

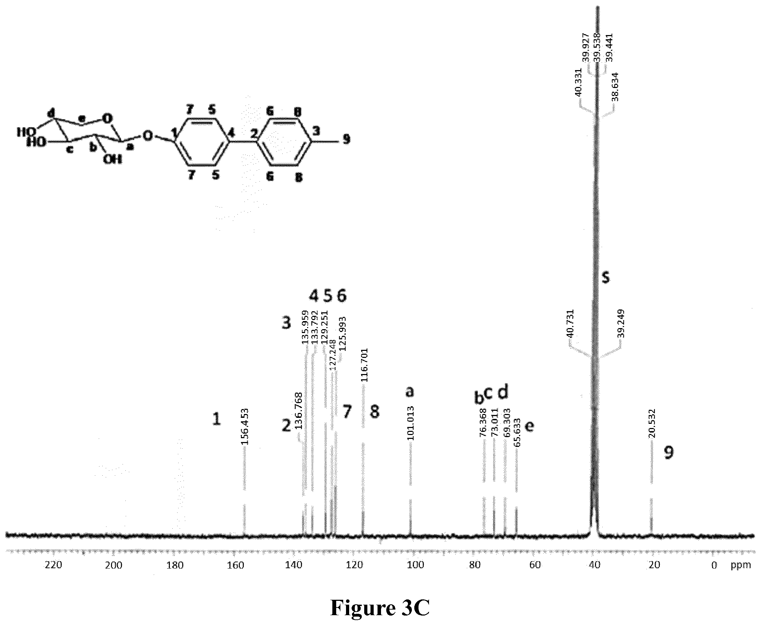

The crude product was purified by column chromatography on silica gel and gave the product as a white powder in a 54.5% yield. Figures 3A and 3B show the ¹H NMR spectrum of 2-(4'-methylbiphenyl-4-yloxy) tetrahydro-2*H*-pyran-3,4,5-triol. Figures 3C shows the ¹³C NMR spectra of 2-(4'-methylbiphenyl-4-yloxy) tetrahydro-2*H*-pyran-3,4,5-triol. In the spectra, the peaks in the range of δ∼3.00-5.50 ppm correlate with the hydrogen atoms of the hydroxyl and tetrahydrofuran moieties. Since there is iodine in the starting material, i.e., 2-(4-iodophenoxy) tetrahydro-2*H*-pyran-3,4,5-triol, the hydrogen resonance shifts from δ7.71 to 7.55 ppm. As shown in Figures 3A-3C, it is confirmed that the desired product was obtained
¹H NMR (200 MHz, CD₆SO): δ7.55 (d, *J* = 8.6Hz, 2H), 7.49 (d, *J* =8.0Hz, 2H), 7.23 (d, *J* =7.8Hz, 2H), 7.06 (d, *J* = 8.8Hz, 2H), 2.32 (s, 3H). ¹³C NMR (50 MHz, CD₆SO): δ156.45, 136.77, 135.96, 133.79, 129.25, 127.25, 125.99, 116.70, 101.01, 76.37, 69.30, 65.63, 20.53. Mass Calcd for C₁₈H₂₀O₅: 316.13.

### Example 4

### 2-(4'-Methoxybiphenyl-4-yloxy) tetrahydro-2H-pyran-3,4,5-triol (Compound 3):

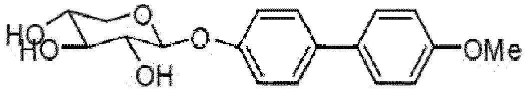

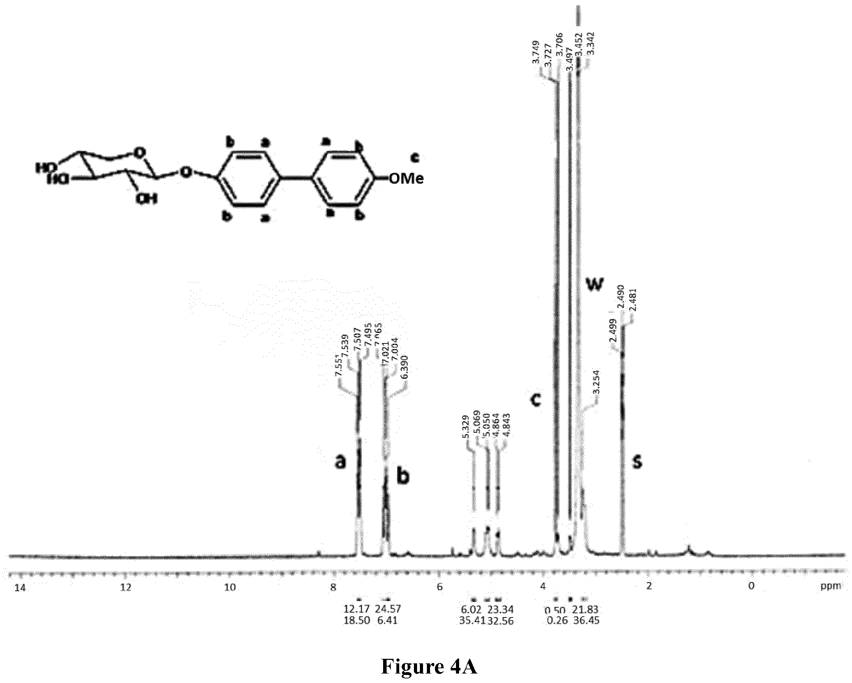

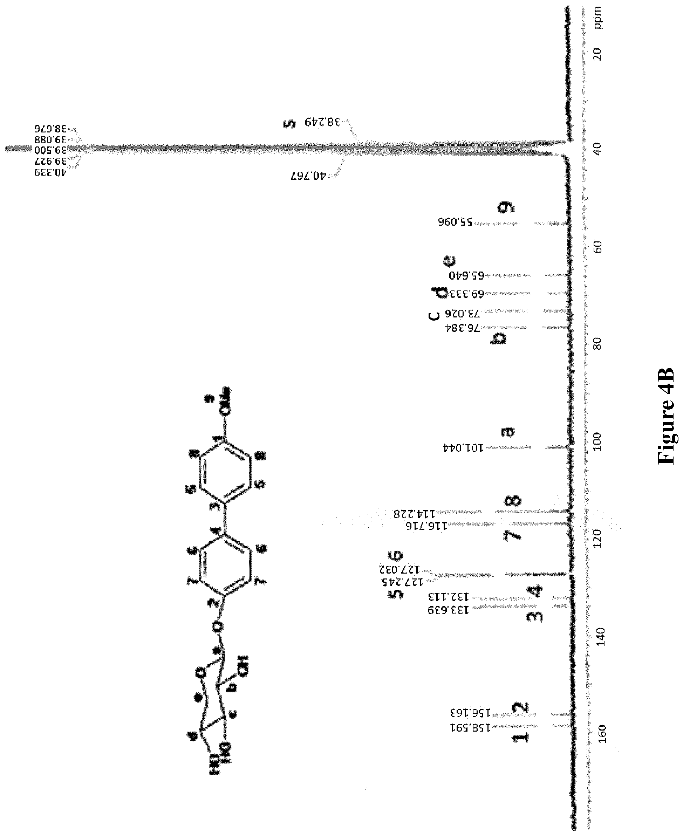

The crude product was purified by column chromatography on silica gel to obtain the product as a white powder in a 78.6% yield. Figures 4A and 4B show the ¹H NMR and ¹³C NMR spectrum of 2-(4'-methoxybiphenyl-4-yloxy) tetrahydro-2*H*-pyran-3,4,5-triol, respectively. In the spectra, the peaks in the range of δ∼3.00-5.50 ppm correlate with the hydrogen atoms of the hydroxyl and tetrahydrofuran moieties. Since there is iodine in the starting material, i.e., 2-(4-iodophenoxy)tetrahydro-2*H*-pyran-3,4,5-triol, the chemical shift of the hydrogen atoms shifts from δ7.71 to 7.54 ppm. As shown in Figures 4A and 4B, it is confirmed that the desired product was obtained.
¹H NMR (200 MHz, CD₆SO): δ7.54 (d, *J* = 4.0Hz, 2H), 7.47 (d, *J* = 8Hz, 2H), 3.69 (s, 3H. ¹³C NMR (50 MHz, CD₆SO): δ158.59, 156.16, 133.64, 127.25, 127.03, 116.72, 114.23, 101.04, 76.39, 73.03, 69.33, 65.64, 55.10. Mass Calcd for C₁₈H₂₀O₆: 316.13.

### Example 5

### 2-(2'-Methylbiphenyl-4-yloxy)tetrahydro-2H-pyran-3,4,5-triol (Compound 4):

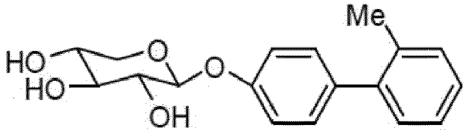

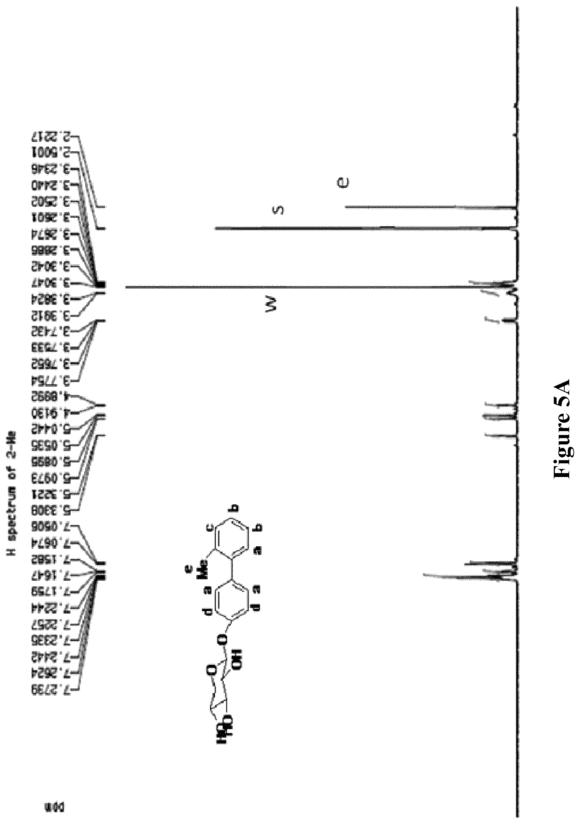

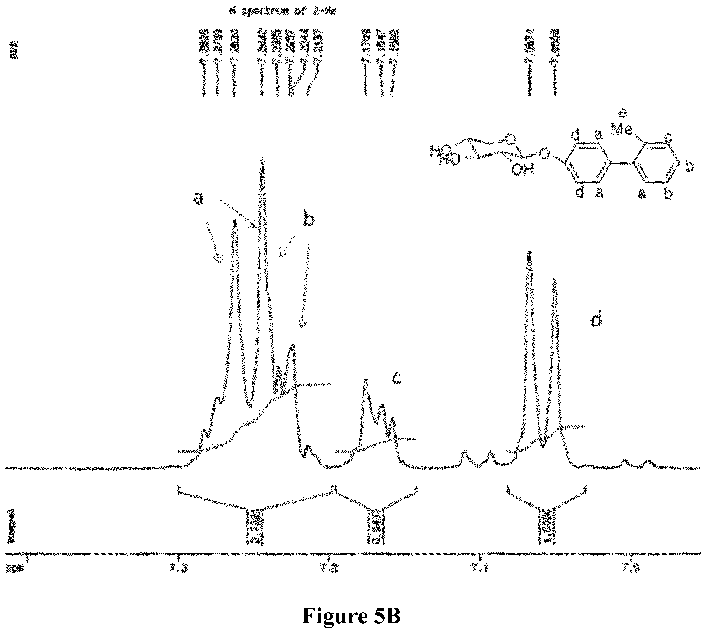

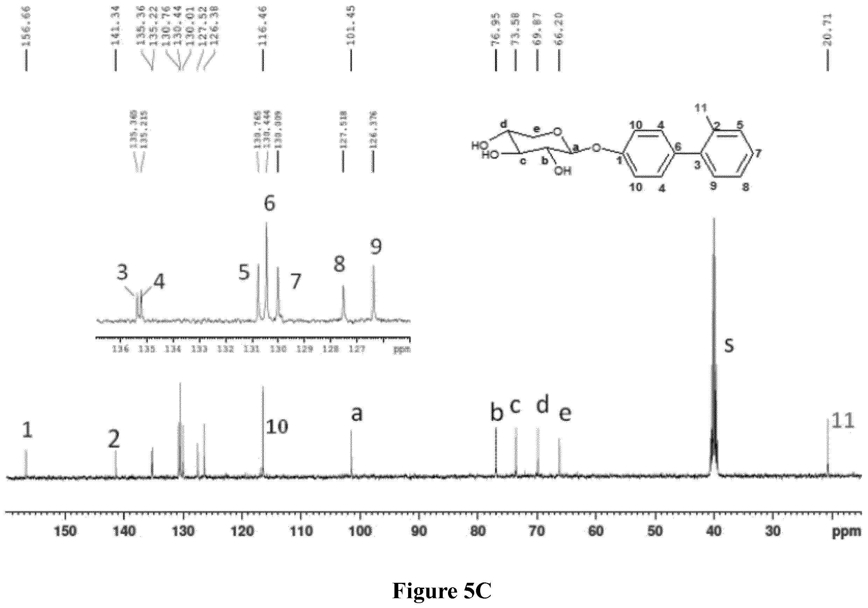

The crude product was purified by column chromatography on silica gel to give the product as a white powder in a 51.8% yield. Figures 5A and 5B show the ¹H NMR spectra of 2-(2'-methylbiphenyl-4-yloxy) tetrahydro-2*H*-pyran-3,4,5-triol. Figure 5C show the ¹³C NMR spectrum of 2-(2'-methylbiphenyl-4-yloxy) tetrahydro-2*H*-pyran-3,4,5-triol. In the spectra, the peaks in the range of δ∼3.00-5.50 ppm correlate with the hydrogen atoms of the hydroxyl and tetrahydrofuran moieties. Since there is iodine in the starting material, i.e., 2-(4-iodophenoxy)tetrahydro-2*H*-pyran-3,4,5-triol, the resonance of the hydrogen atom shifted fromδ7.71 to 7.25 ppm. As shown in Figures 5A-5C, it is confirmed that the desired product was obtained.
¹H NMR (400 MHz, CD₆SO): δ7.25 (d , *J* =7.28Hz, 3H), 7.23 (t , *J* =7.4Hz, 2H), 7.16 (d, *J* =4.48Hz, 2H), 7.06(d, *J* =4.48Hz.2H), 2.22(s, 3H). ¹³C NMR (100 MHz, CD₆SO): δ156.66, 141.34, 135.36, 135.22, 130.76, 130.44, 130.01, 127.52, 126.38, 116.46, 101.45, 76.95, 73.58, 69.87, 66.20, 20.71. Mass Calcd for C₁₈H₂₀O₅: 316.13.

### Example 6

### 2-(2'-Chlorobiphenyl-4-yloxy)tetrahydro-2H-pyran-3,4,5-triol (Compound 5):

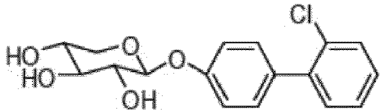

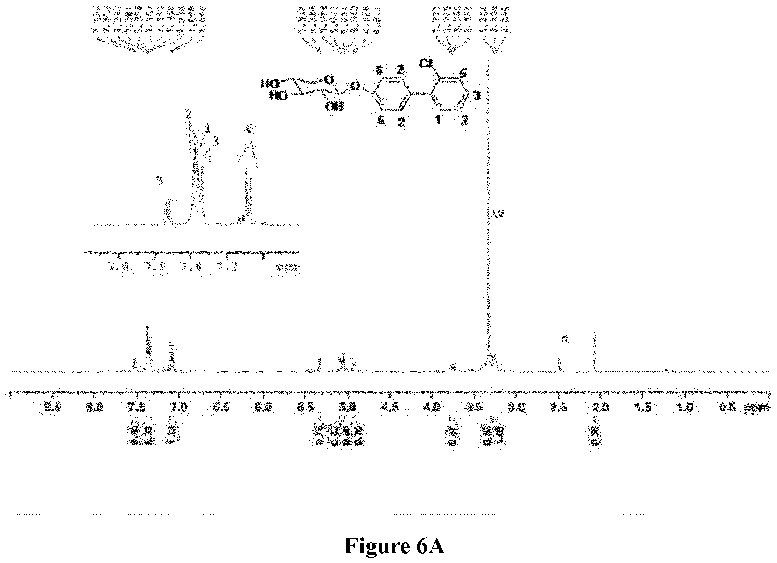

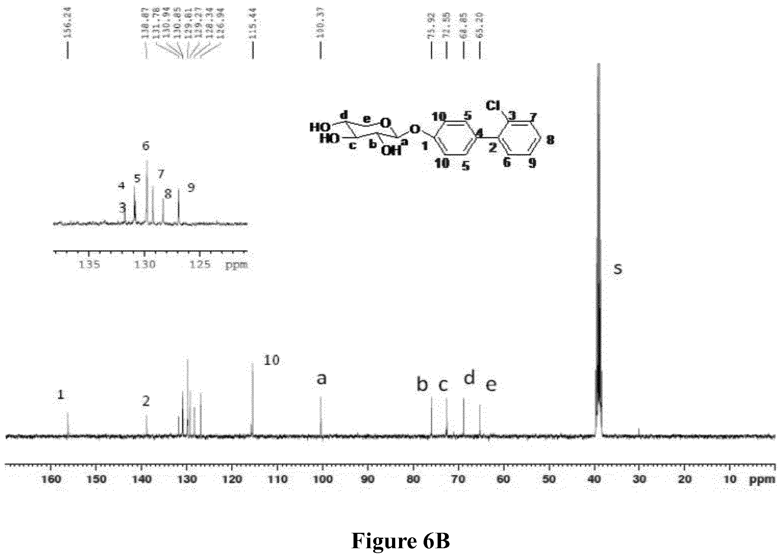

The crude product was purified by column chromatography on silica gel to give the product in a 59.3% yield as a white powder. As shown in Figures 6A and 6B providng the ¹H NMR and ¹³C NMR spectra of 2-(2'-chlorobiphenyl-4-yloxy)tetrahydro-*2H*-pyran-3,4,5-triol, respectively. The peaks in the range of δ∼3.00-5.50 ppm correlate with the hydrogen atoms of the hydroxyl and tetrahydrofuran moieties. Since there is iodine in the starting material, i.e., 2-(4-iodophenoxy)tetrahydro-2*H*-pyran-3,4,5-triol), the resonance for the hydrogen atom shifted from δ7.71 to 7.39 ppm. A shown in Figures 6A and 6B, it is confirmed that the desired product was obtained
¹H NMR (400 MHz, CD₆SO): δ7.53(d, 6.8Hz, 1H), 7.39(d, *J* = 4.8Hz, 2H), 7.37(d, *J* =4.4Hz, 1H), 7.35(t, 6.8Hz, 2H), 7.08(d, *J* = 8.8 MHz, 2H). ¹³C NMR (100 MHz, CD₆SO): δ156.24, 138.87, 131.78, 130.94, 130.85, 129.81, 129.27, 128.34, 126.94, 115.44, 100.37, 75.92, 72.55, 72.55, 68. Mass Calcd for C₁₇H₁₇ClO₅: 336.13.

### Example 7

### 2-(2'-Methoxybiphenyl-4-yloxy) tetrahydro-2H-pyran-3,4,5-triol (Compound 6):

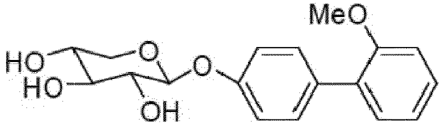

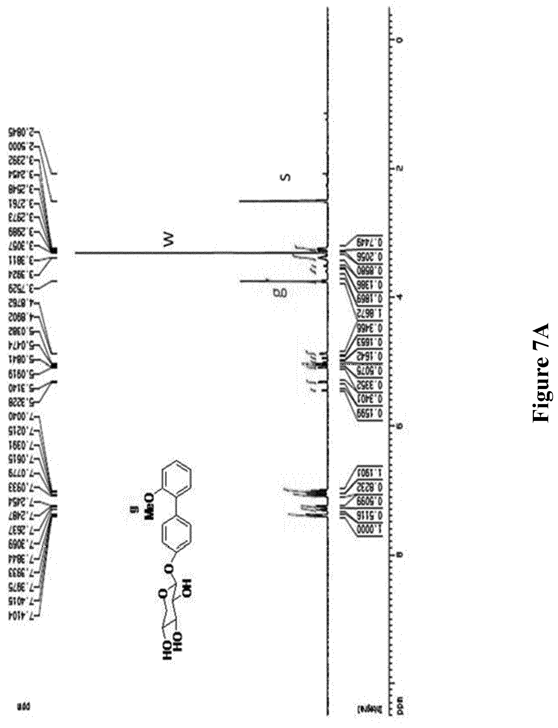

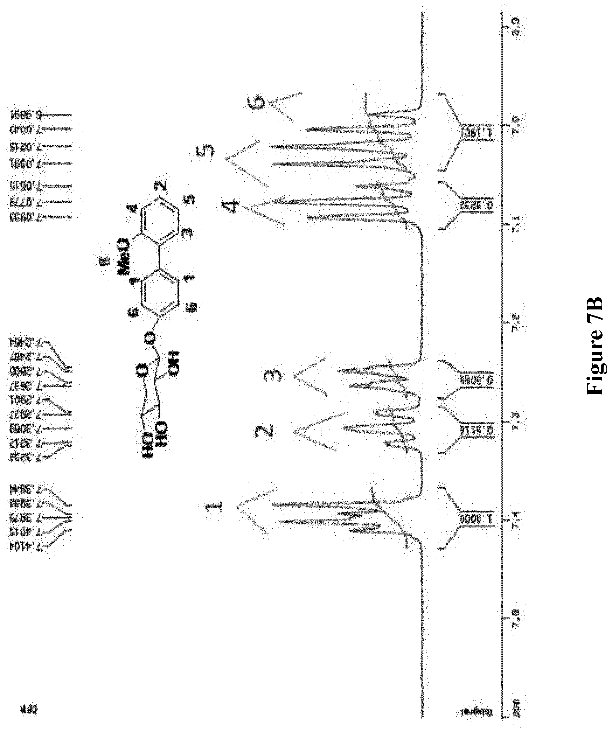

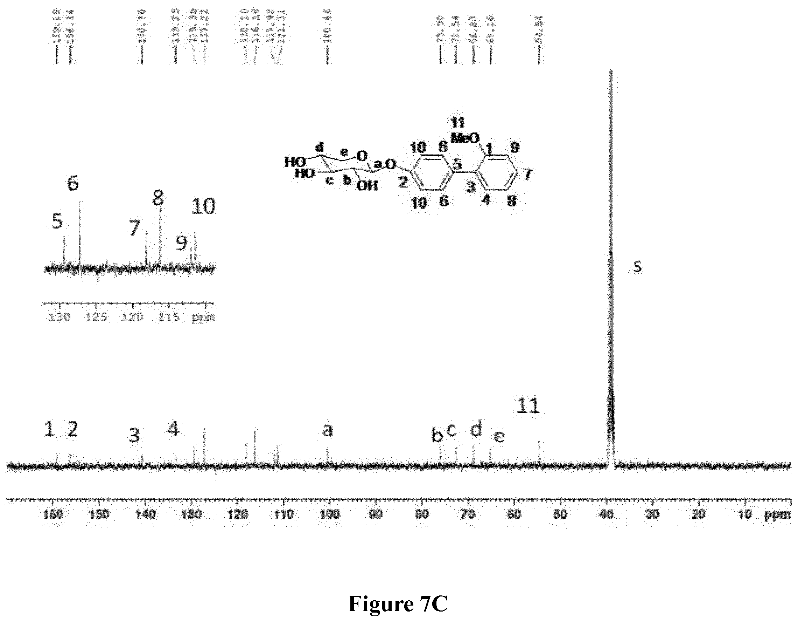

The crude product was purified by column chromatography on silica gel to give the product as a white powder in a 60.8% yield. Figures 7A and 7B show the ¹H NMR spectra of 2-(2'-methoxybiphenyl-4-yloxy) tetrahydro-2*H*-pyran-3,4,5-triol. Figures 7C show the ¹³C NMR spectrum of 2-(2'-methoxybiphenyl-4-yloxy) tetrahydro-2*H*-pyran-3,4,5-triol. In the spectra, the peaks in the range of δ3∼.00-5.50 ppm correlate with the hydrogen atoms of the hydroxyl and tetrahydrofuran moieties. Since there is iodine in the starting material, i.e., 2-(4-iodophenoxy) tetrahydro-2*H*-pyran-3,4,5-triol, the resonance for the hydrogen atom shifts from δ7.71 to 7.39 ppm. As shown in Figures 7A-7C, it is confirmed that the desired product was obtained.
¹H NMR (400 MHz, CD₆SO): δ7.39 (d, 6.84Hz, 2H), 7.32 (t, 5.24Hz, 1H), 7.25(d, 7.32Hz, 1H), 7.09(d, 6.16Hz, 1H), 7.04(d, 7.04Hz, 1H), 7.00(d,5.96Hz, 2H). ¹³C NMR (100 MHz, CD₆SO): δ159.19, 156.34, 140.70, 133.25, 129.35, 127.22, 118.10, 116.18, 111.92, 111.32, 100.46, 75.90, 72.54, 68.83, 65.16, 54.54. Mass Calcd for C₁₈H₂₀O₆: 332.13

### Example 8

### 2-(3'-Methylbiphenyl-4-yloxy) tetrahydro-2H-pyran-3,4,5-triol (Compound 7):

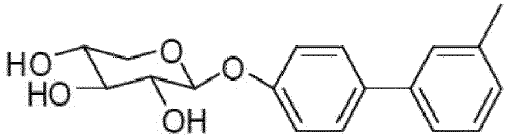

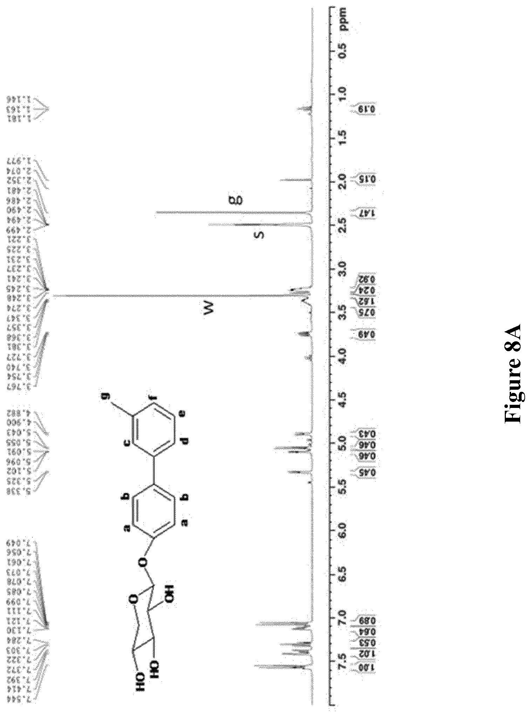

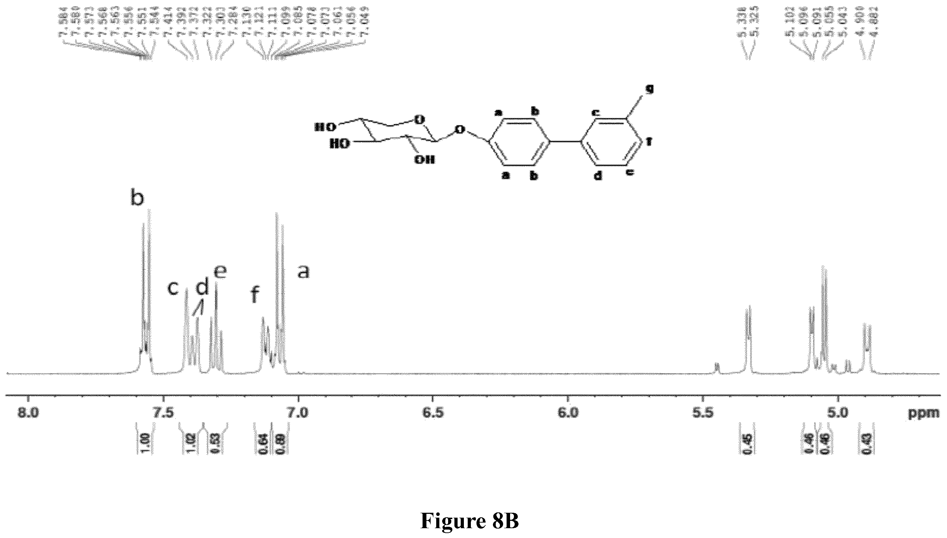

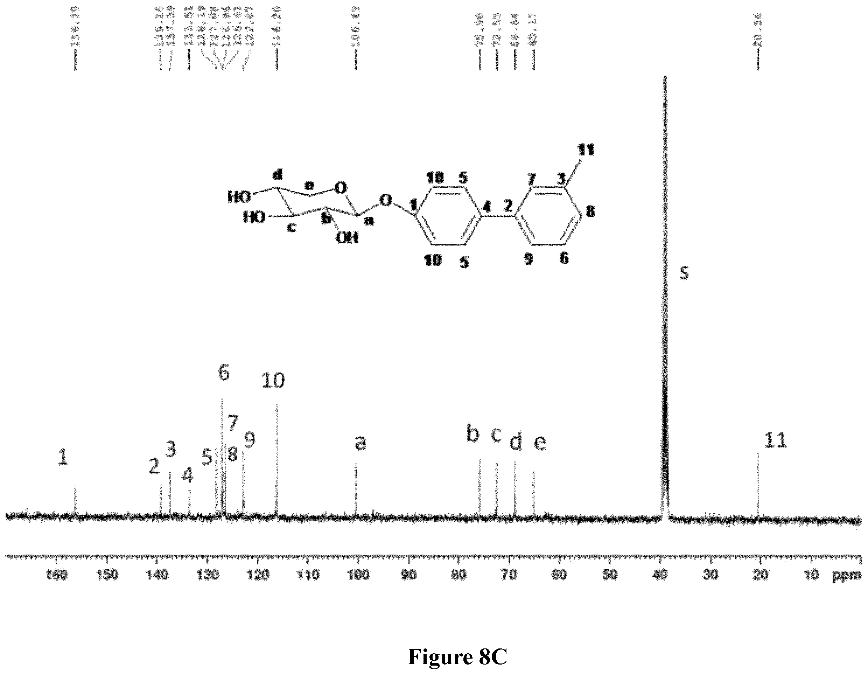

The crude product was purified by column chromatography on silica gel to give the product as a white powder in an 87.5% yield. Figures 8A and 8B show the ¹H NMR spectra of 2-(3'-methylbiphenyl-4-yloxy) tetrahydro-2*H*-pyran-3,4,5-triol. Figures 8C shows the ¹³C NMR spectrum of 2-(3'-methylbiphenyl-4-yloxy) tetrahydro-2*H*-pyran-3,4,5-triol. In the spectra, the peaks in the range of δ∼3.00 -5.50 ppm correlate with the hydrogen atoms of the hydroxyl and tetrahydrofuran moieties. Since there is iodine in the starting material, i.e., 2-(4-iodophenoxy)tetrahydro-2*H*-pyran-3,4,5-triol, the resonance for the hydrogen shifts from δ7.71 to 7.57 ppm. As shown in Figures 8A-8C, it is confirmed that the desired product was obtained.
¹H NMR (400 MHz, CD₆SO): δ 7.57(d, 11.6Hz, 2H), 7.41 (s, 1H), 7.30(t, 6.4Hz, 1H), 7.12(d, 8H, 1H), 7.05(d, 2.8Hz, 2H), 2.49(s,3H). ¹³C NMR (100 MHz, CD₆SO): δ159.19, 139.16, 137.39, 133.51, 128.19, 127.8, 126.41, 122.87, 116.20, 100.49, 75.90, 72.55, 68.84, 65.17, 20.56. Mass Calcd for C₁₈H₂₀O₅: 316.12

### Example 9

### 2-(3'-Methoxybiphenyl-4-yloxy) tetrahydro-2H-pyran-3,4,5-triol (Compound 8):

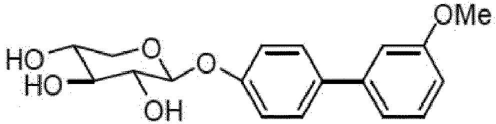

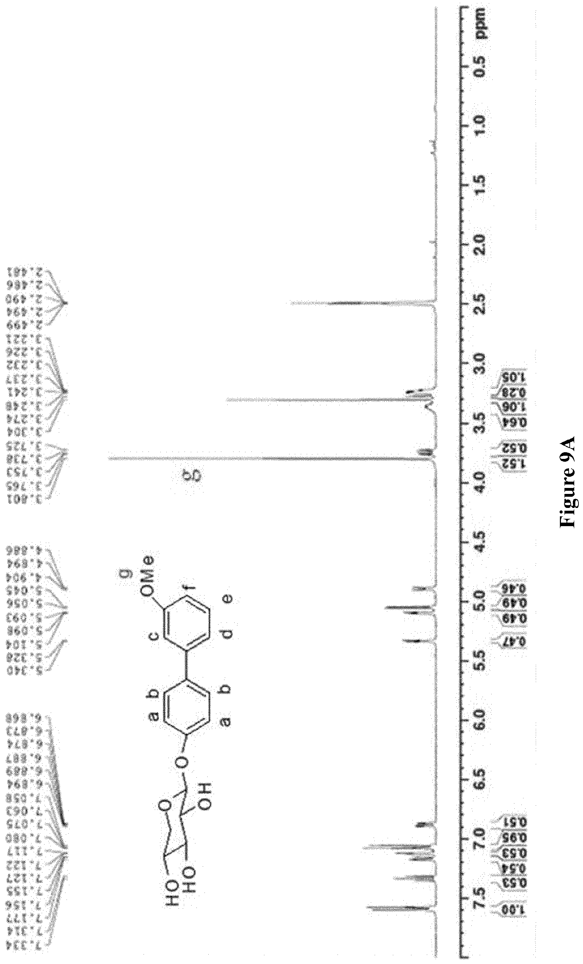

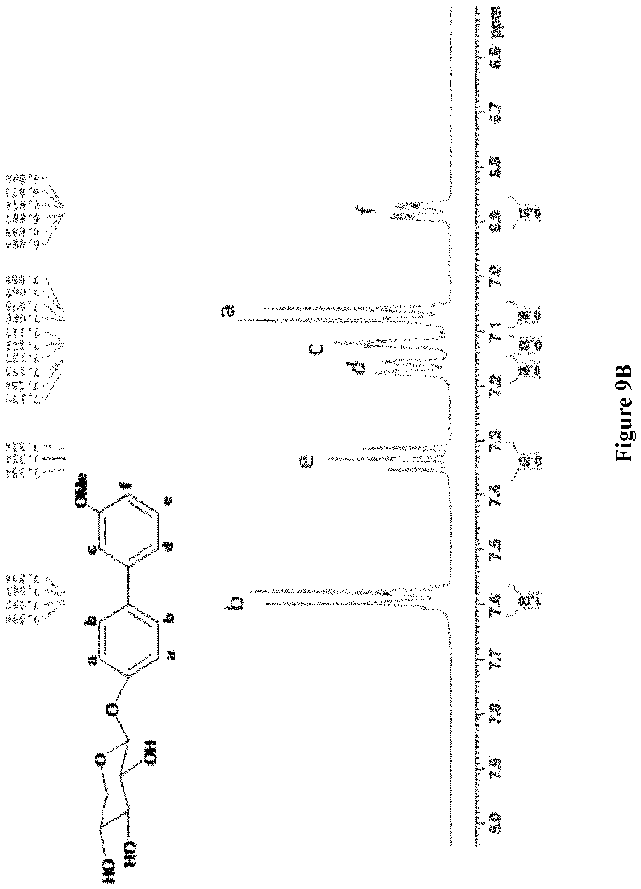

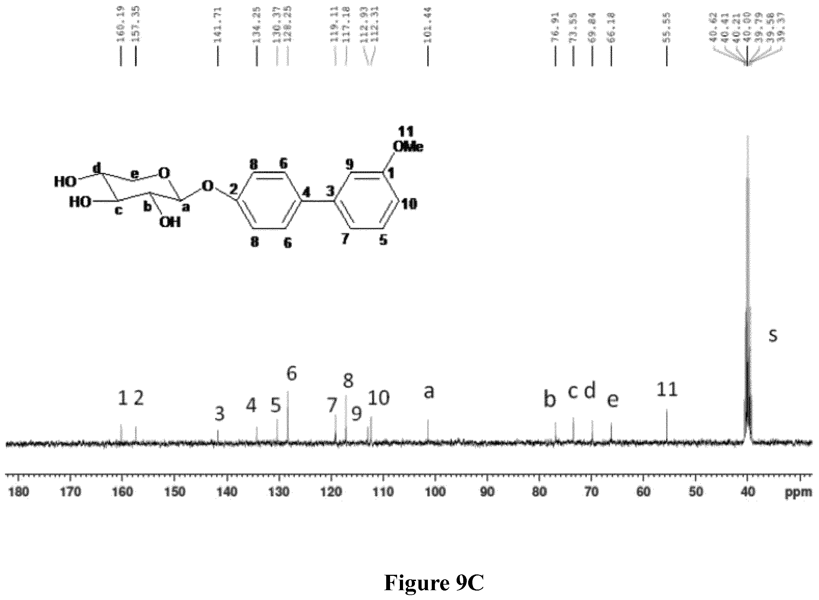

The crude product was purified by column chromatography on silica gel to give the product as a white powder in a 92.0% yield. Figures 9A and 9B show the ¹H NMR spectra of 2-(3'-methoxybiphenyl-4-yloxy)tetrahydro-2*H*-pyran-3,4,5-triol. Figure 9C shows the ¹³C NMR spectrum of 2-(3'-methoxybiphenyl-4-yloxy)tetrahydro-*2H*-pyran-3,4,5-triol. In the spectra, the peaks in the range of δ∼3.00-5.50 ppm correlate with the hydrogen atoms of the hydroxyl and tetrahydrofuran moieties. Since there is iodine in the starting material, i.e., 2-(4-iodophenoxy)tetrahydro-2*H*-pyran-3,4,5-triol, the resonance for the hydrogen atom shifts fromδ7.71 to 7.59 ppm. As shown in Figures 9A-9C, it is confirmed that the desired product was obtained.
¹H NMR (400 MHz, CD₆SO): δ7.59(d, *J* = 3.6Hz, 2H), 7.34(t, *J* = 8.0Hz, 1H), 7.17(d, *J* = 8.8Hz, 1H), 7.12(s, 1H), 7.07(D, *J* = 8.8Hz,2H), 6.88(d, *J* = 8.4, 1H), 3.75(s, 3H). ¹³C NMR (100 MHz, CD₆SO): δ159.18, 156.34, 140.70, 133.25, 129.34, 127.21, 118.09, 116.18, 111.91, 111.30, 100.46, 75.89, 72.53, 68.83, 65.16, 54.54. Mass Calcd for C₁₈H₂₀O₆: 332.13

### Example 10

### 2-(3'-Chlorobiphenyl-4-yloxy) tetrahydro-2H-pyran-3,4,5-triol (Compound 9):

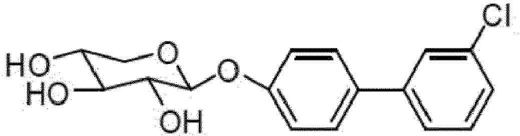

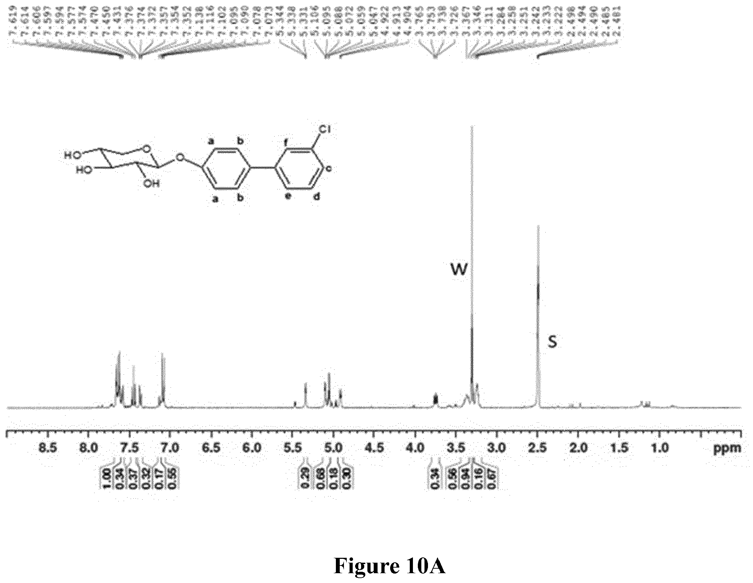

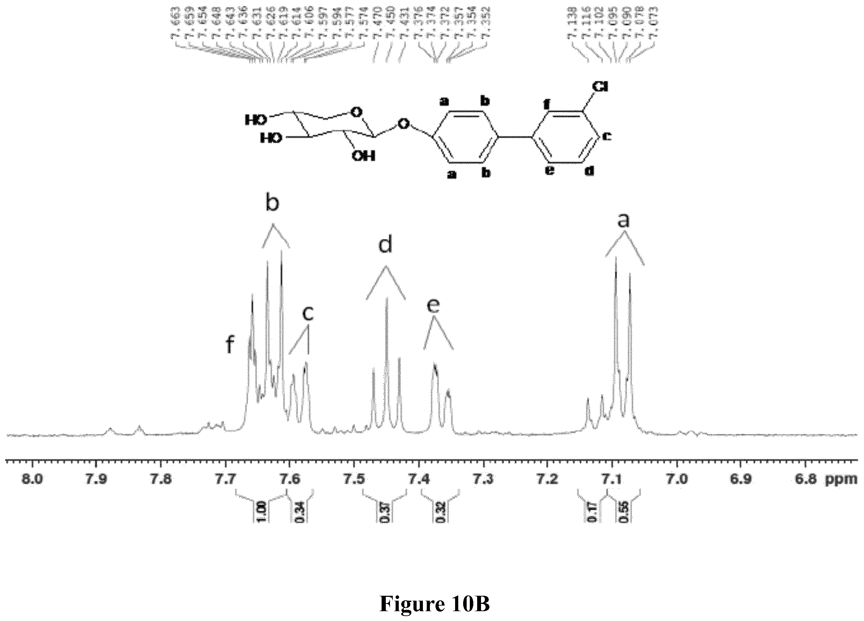

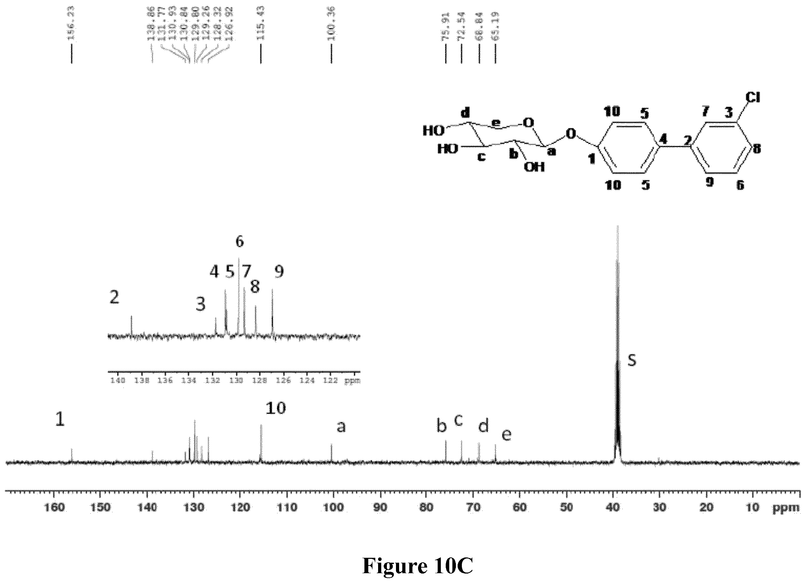

The crude product was purified by column chromatography on silica gel to give the product as a white powder in a 77.2% yield. Figures 10A and 10B show the ¹H NMR spectra of 2-(3'-chlorobiphenyl-4-yloxy) tetrahydro-*2H*-pyran-3,4,5-triol. Figure 10C shows the ¹³C NMR spectrum of 2-(3'-methoxybiphenyl-4-yloxy)tetrahydro-*2H*-pyran-3,4,5-triol. In the spectra, the peaks in the range of δ∼3.00-5.50 ppm correlate with the hydrogen atoms of the hydroxyl and tetrahydrofuran moieties. Since there is iodine in the starting material, i.e., 2-(4-iodophenoxy) tetrahydro-2*H*-pyran-3,4,5-triol, the resonance for the hydrogen atom shifts fromδ7.71 to 7.63 ppm. As shown in Figures 10A-10C, it is confirmed that the desired product was obtained.
¹H NMR (400 MHz, CD₆SO): δ7.66(s, *J* =8.0Hz, 1H), 7.63(d, *J* = 8.0Hz, 2H), 7.59(d, *J* =9.8Hz, 1H), 7.45(t, *J* = 8.0 Hz 1H), 7.35(d, *J* =2 Hz, 1H), 7.10(d, *J* = 11.6 Hz, 2H). ¹³C NMR (100 MHz, CD₆SO): δ156.23, 138.86, 131.77, 130.93, 130.84, 129.80, 129.26, 128.31, 126.92, 115.43, 110.36, 75.91, 72.54, 68.86, 65.19. Mass Calcd for C₁₇H₁₇ClO₅: 336.08.

### Example 11

### 2-(3',5'-Dimethylbiphenyl-4-yloxy)tetrahydro-2H-pyran-3,4,5-triol (Compound 10):

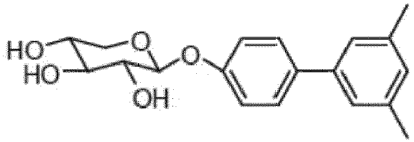

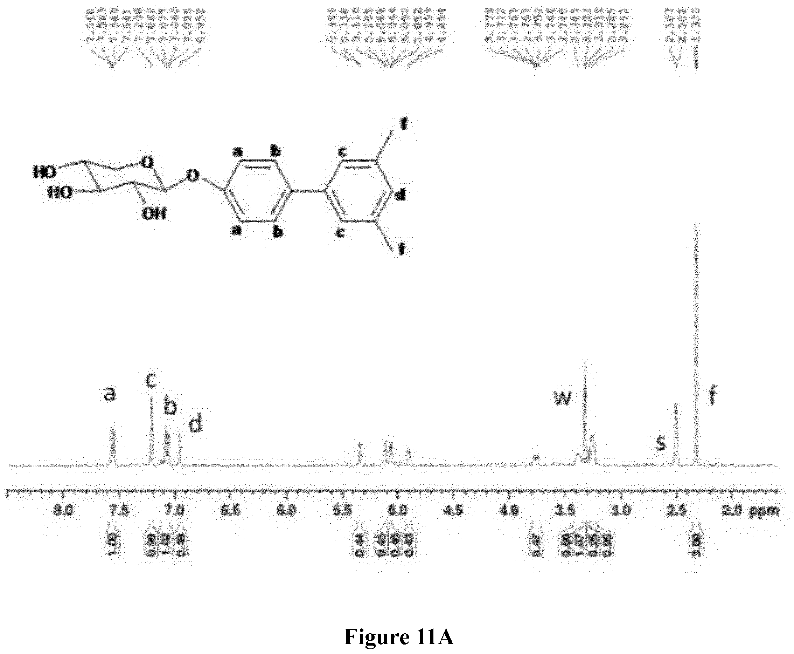

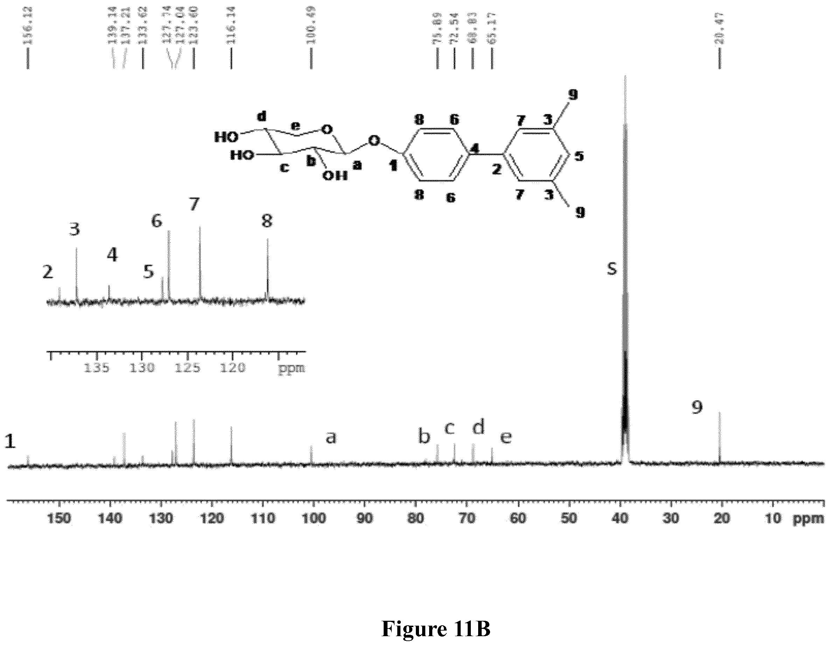

The crude product was purified by column chromatography on silica gel to give the product as a white powder in a 98.0% yield. Figures 11A and 11B show the ¹H NMR and ¹³C NMR spectra of 2-(3',5'-dimethylbiphenyl-4-yloxy)tetrahydro-2*H*-pyran-3,4,5-triol, respectively. In the spectra, the peaks in the range of δ∼3.00-5.50 ppm correlate with the hydrogen atoms of the hydroxyl and tetrahydrofuran moieties. Since there is iodine in the starting material, i.e., 2-(4-iodophenoxy)tetrahydro-2*H*-pyran-3,4,5-triol, the resonance for the hydrogen atom shifts from δ7.71 to 7.07 ppm. As shown in Figures 11A and 11B, it is confirmed that the desired product was obtained.
¹H NMR (400 MHz, CD₆SO): δ7.5 6(d , *J* = 8.8Hz, 2H), 7.2 1(s, 2H), 7.07(d, *J* = 8.8Hz, 2H), 6.95(s , 1H), 2.32(s, 6H). ¹³C NMR (100 MHz, CD₆SO): δ156.12, 139.14, 137.21, 133.62, 127.74, 127.60, 123.60, 116.14, 110.49, 75.89, 72.54, 68.83, 65.17, 20.47. Mass Calcd for C₁₉H₂₂O₅: 330.15.

## Claims

1. A xylose derivative having the general formula I: 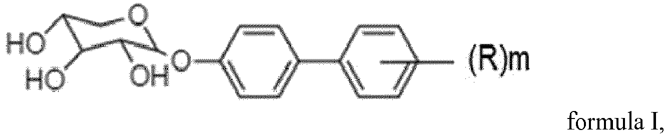 wherein the m for (R)m is an integer from 1 to 4; R is CH₃, OCH₃, or a halogen atom.

2. The xylose derivative of claim 1, which is one selected from the group consisting of the following: 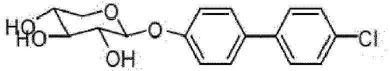 2-(4'-chlorobiphenyl-4-yloxy) tetrahydro-2*H*-pyran-3,4,5-triol (Compound 1); 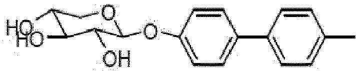 2-(4'-methylbiphenyl-4-yloxy) tetrahydro-2*H*-pyran-3,4,5-triol (Compound 2); 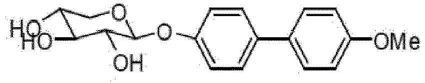 2-(4'-methoxybiphenyl-4-yloxy) tetrahydro-2*H*-pyran-3,4,5-triol (Compound 3); 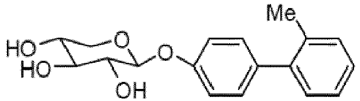 2-(2'-methylbiphenyl-4-yloxy)tetrahydro-2*H*-pyran-3,4,5-triol (Compound 4); 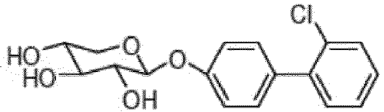 2-(2'-chlorobiphenyl-4-yloxy)tetrahydro-2*H*-pyran-3,4,5-triol (Compound 5); 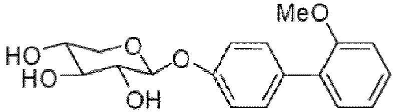 2-(2'-methoxybiphenyl-4-yloxy) tetrahydro-2*H*-pyran-3,4,5-triol (Compound 6); 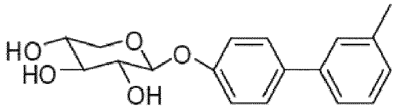 2-(3'-methylbiphenyl-4-yloxy) tetrahydro-2*H*-pyran-3,4,5-triol (Compound 7); 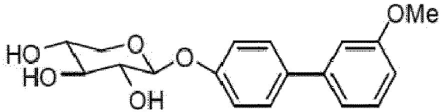 2-(3'-methoxybiphenyl-4-yloxy) tetrahydro-2*H*-pyran-3,4,5-triol (Compound 8); 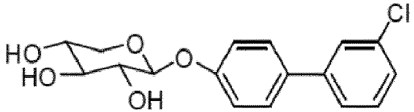 2-(3'-chlorobiphenyl-4-yloxy) tetrahydro-2*H*-pyran-3,4,5-triol (Compound 9); and 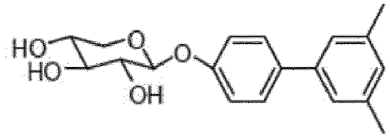 2-(3',5'-dimethylbiphenyl-4-yloxy)tetrahydro-*2H*-pyran-3,4,5-triol (Compound 10).

3. A method for synthesis of a xylose derivative of claim 1, comprising the steps of:
protecting a xylose with a protective group, followed by incorporating to a halogen atom as a leaving group;
removing the protective group and using a water-soluble ligand to carry out Suzuki cross-coupling reaction with a palladium catalyst in a water solution to synthesize the xylose derivative.

4. The method of claim 3, wherein the halogen atom is an iodine atom.

5. The method of claim 3, wherein the water-soluble ligand is recycled.

6. The method of claim 3, wherein the palladium catalyst is recycled.

7. The method of claim 3, wherein the palladium catalyst is PdCl₂(NH₃)₂, and the water-soluble ligand is a cationic 2,2'-bipyridyl ligand.

8. The method of claim 7, wherein a cationic 2,2'-bipyridyl ligand is dissolved in water to form a solution A; and PdCl₂(NH₃)₂ is dissolved in water to form a solution B; and adding the solution A to the solution B to generate a turbid solution that eventually cleared to obtain a water-soluble palladium catalyst.

9. The method of claim 3, wherein the Suzuki cross-couplings reaction is carried out at a temperature no higher than 100°C.
